(19) Europäisches Patentamt
European Patent Office
Office européen des brevets

(11) EP 1 117 420 B1

(12) EUROPEAN PATENT SPECIFICATION

(45) Date of publication and mention of the grant of the patent:
**05.06.2002 Bulletin 2002/23**

(21) Application number: **99948935.4**

(22) Date of filing: **23.09.1999**

(51) Int Cl.[7]: **A61K 38/09**, A61K 47/40, A61P 5/06

(86) International application number:
**PCT/EP99/07389**

(87) International publication number:
**WO 00/18423 (06.04.2000 Gazette 2000/14)**

# (54) PHARMACEUTICAL COMPOSITIONS BASED ON ALPHA-CYCLODEXTRIN FOR THE ORAL ADMINISTRATION OF LH-RH ANALOGUES

PHARMAZEUTISCHE ZUSAMMENSETZUNGEN BASIEREND AUF ALPHA-CYCLODEXTRIN ZUR ORALEN VERABREICHUNG VON LH-RH ANALOGEN

COMPOSITIONS PHARMACEUTIQUES A BASE D'ALPHA-CYCLODEXTRINE DESTINEES A L'ADMINISTRATION ORALE D'ANALOGUES DE LH-RH

(84) Designated Contracting States:
**AT BE CH CY DE DK ES FI FR GB GR IE IT LI LU MC NL PT SE**
Designated Extension States:
**AL LT LV SI**

(30) Priority: **30.09.1998 EP 98402403**

(43) Date of publication of application:
**25.07.2001 Bulletin 2001/30**

(73) Proprietor: **LABORATOIRE THERAMEX S.A.**
**98000 Monaco (MC)**

(72) Inventors:
- **DELANSORNE, Rémi**
  **F-06000 Nice (FR)**
- **BONNET, Paule**
  **F-06500 Menton (FR)**
- **PARIS, Jacques**
  **F-06100 Nice (FR)**

(74) Representative: **Nevant, Marc et al**
**Cabinet Beau de Loménie,**
**158, rue de l'Université**
**75007 Paris Cedex 07 (FR)**

(56) References cited:
**EP-A- 0 308 181**
**EP-A- 0 839 525**
**EP-A- 0 842 946**
**WO-A-95/07076**
**US-A- 4 659 696**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 117 420 B1

## Description

The present invention relates to the pharmaceutical field. More specifically, the invention relates to the use of α-cyclodextrin or derivatives thereof for the preparation of pharmaceutical compositions for the oral administration of LH-RH (luteinizing hormone - releasing hormone) peptide analogues. The invention also relates to oral pharmaceutical compositions containing LH-RH peptide analogues in combination with α-cyclodextrin.

Natural and modified cyclodextrins (CDs) are well known ingredients used in a large variety of pharmaceutical preparations taking advantage of one or several of their properties relating to drug solubilization and stabilization (Loftsson and Brewster, 1996, *J. Pharm. Sci.*, **85**(10): 1017-1025) or to overall improvement of *in vivo* drug delivery (Rajewski and Stella, 1996, *J. Pharm. Sci.*, **85**(11): 1142-1169). CDs are cyclic oligosaccharides containing at least 6 α-D-(+)(+)-glucopyranose units attached by α(1-4) glucoside bonds (Nash, *Handbook of Pharmaceutical Excipients,* ed. by Wade and Weller, 1994, American Pharmaceutical Association, Washington, and The Pharmaceutical Press, London, pp 145-148); the three most common CDs are α-, β- and γ-CD which consist of 6, 7 and 8 sugar units, respectively. Numerous derivatives of each type of CD can be obtained by random or controlled modifications of one, several or all free hydroxyl groups of the sugar moities.

LH-RH is a neurohormone produced by hypothalamic neurons and secreted in the pituitary portal vasculature to stimulate the release of luteinizing hormone (LH) and follicle stimulating hormone (FSH) by the pituitary gland. In turn, LH and FSH regulate the endocrine and germinal functions of the ovary in the female and of the testis in the male. LH-RH is a peptide of the following structure: pGlu-His-Trp-Ser-Tyr-Gly-Leu-Arg-Pro-Gly-$NH_2$. Numerous normal or reduced-size, linear or cyclic peptide analogues of LH-RH incorporating natural, unusual or chemically-modified amino-acids have been synthesized over the years to yield potent agonist or antagonistic properties (Karten and Rivier, 1986, *Endocr. Rev.*, **7**(1): 44-66; Dutta, 1988, *Drugs of the Future,* **13**(8): 761-787; Kutscher et al., 1997, *Angew. Chem. Int. Ed. Engl.,* **36**: 2148-2161). Due to their total or partial peptide structure, however, all these analogues show poor oral bioavailability and bioactivity.

To date, only non-oral administration of LH-RH peptide analogues, has been reported. For example, Matsubara et al. (1996, J. Pharm. Sci., **84**(11) : 1295-1300) describe a nasal formulation of buserelin, based on dimethyl-p-CD, with improved bioavailability.

EP-A-0 308 181 discloses a preparation for non-enteral Trans-mucosal drug delivery (including oral-mucosal) comprising a pharmaceutically active agent such as LH-RH and an absorption enhancer such as α-cyclodextrin.

EP-A-0839525 discloses an oral preparation of a sustained release formulation comprising a polymer of lactic acid and LH-RH agonists and antagonists. Statilisers such as α-cyclodextrin and a derivative thereof may be added.

There is therefore a need, for the patients' comfort, to provide formulations which enable the oral administration of LH-RH peptide analogues.

It has now surprisingly been found that α-CD or its derivatives, enhance the biological activity of LH-RH peptide analogues when orally administered.

Thus, according to one of its feature, the invention relates to the use of α-cyclodextrin or derivatives thereof for the preparation of pharmaceutical compositions for the oral administration of LH-RH peptide analogues.

Examples of LH-RH peptide analogues which can be used within the scope of the invention include those described in International patent applications WO 98/21229 and WO 98/55505, the content of which Is incorporated by reference, as well as standard agonists and antagonists of LH-RH, such as for example buserelin, nafarelin, leuprorelin, goserelin, histrelin, triptorelin, deslorelin, lutrelin, avorelin, cetrorelix, antide, ganirelix, azaline B, antarelix, detirelix, ramorelix, teverelix or abarelix.

Preferably, these peptide analogues have the formula (SEQ ID N° : 1):

A1-A2-A3-A4-A5-A6-A7-A8-Pro-Z (A)

in which:

- A1 is pGlu ; D-pGlu ; Sar; AcSar ; Pro or a derivative thereof such as AcPro, ForPro, OH-Pro, Ac-OH-Pro, dehydro-Pro or Ac-dehydro-Pro ; Ser; D-Ser ; Ac-D-Ser ; Thr; D-Thr ; Ac-D-Thr; or an aromatic D-amino acid which may be acylated, such as D-Phe, D-HPhe, D-Tyr, D-HTyr, D-Trp, D-2MeTrp, D-Nal, D-1Nal, D-diphenyl-Ala, D-Bal, D-Pal, D-4Pal or D-Qal, where D-Phe, D-HPhe, D-Tyr, D-HTyr, and D-Trp may be substituted by one or more halogens, ($C_1$-$C_4$)alkyl, ($C_1$-$C_4$)alkoxy, nitro or trifluoromethyl groups;
- A2 is a direct bond ; His ; or an aromatic D-amino acid such as D-Phe, D-HPhe, D-Tyr, D-HTyr, D-Trp, D-2MeTrp, D-Nal, D-1Nal, D-diphenyl-Ala, D-Bal, D-Pal, D-4Pal or D-Qal, where D-Phe, D-HPhe, D-Tyr, D-HTyr and D-Trp may be substituted by one or more halogens, ($C_1$-$C_4$)alkyl, ($C_1$-$C_4$)alkoxy, nitro or trifluoromethyl groups;
- A3 is an aromatic L- or D-amino acid such as Phe, HPhe, Tyr, HTyr, Trp, 2MeTrp, Nal, 1Nal, diphenyl-Ala, Bal,

Pal, 4Pal or Qal, where Phe, HPhe, Tyr, HTyr and Trp may be substituted by one or more halogens, $(C_1-C_4)$alkyl, $(C_1-C_4)$alkoxy, nitro or trifluoromethyl groups;

- A4 is Ala, Ser, D-Ser, MeSer, Ser($OBu^t$), Ser(OBzl) or Thr;
- A5 is an aromatic L-amino acid such as Phe, HPhe, Tyr, HTyr, Trp, 2MeTrp, Nal, 1Nal, diphenyl-Ala, Bal, Pal, 4Pal or Qal, where Phe, HPhe, Tyr, HTyr and Trp may be substituted by one or more halogens, $(C_1-C_4)$alkyl, $(C_1-C_4)$alkoxy, nitro or trifluoromethyl groups and/or N-alpha-substituted by a $(C_1-C_4)$alkyl group optionally substituted by one or several fluorine atoms ; or a basic L- or D-amino acid such as Arg, HArg, Orn, Lys, HLys, Cit, HCit, APhe or ACha, where Arg and HArg may be N-substituted by a $(C_1-C_6)$alkyl or a $(C_3-C_6)$cycloalkyl group on one or both nitrogen atoms, and where Orn, Lys, HLys, APhe and ACha may be N-substituted by one or two $(C_1-C_6)$alkyl or $(C_3-C_6)$cycloalkyl groups, or by an aminotriazolyl or a nicotinoyl, isonicotinoyl, 6-methyl-nicotinoyl, glycyl-nicotinoyl, nicotinyl-azaglycyl, furyl, glycyl-furyl, furyl-azaglycyl, pyrazinyl, pyrazinyl-carbonyl, picolinoyl, 6-methyl-picolinoyl, shikimyl, shikimyl-glycyl, Fmoc or Boc group;
- A6 is Gly; (S)-spirolactam-Pro ; D-Pro ; D-Ser ; D-Thr ; D-Cys ; D-Met; D-Asn ; D-Pen ; D-(S-Me)Pen ; D-(S-Et)Pen ; D-Ser($OBu^t$) ; D-Asp($OBu^t$) ; D-Glu($OBu^t$) ; D-Thr($OBu^t$) ; D-Cys($OBu^t$) ; D-Ser($OR_1$) where $R_1$ is a sugar moiety ; an aza-amino acid such as azaGly or azaAla ; D-His which may be substituted on the imidazole ring by a $(C_1-C_6)$alkyl, a $(C_2-C_7)$acyl or a benzyl group ; an aliphatic D-amino acid with a $(C_1-C_8)$alkyl or a $(C_3-C_6)$cycloalkyl side chain such as D-Ala, D-Abu, D-Aib, D-3Aib, D-Val, D-Nva, D-Leu, D-Ile, D-Tle, D-Nle, D-Hol, D-Npg, D-CPa, D-Cpa, D-Cba or D-Cha ; an aromatic D-amino acid such as D-Phe, D-HPhe, D-Tyr, D-HTyr, D-Trp, D-2MeTrp, D-Nal, D-1Nal, D-diphenyl-Ala, D-anthryl-Ala, D-phenanthryl-Ala, D-benzhydryl-Ala, D-fluorenyl-Ala, D-Bal, D-Pal, D-4Pal or D-Qal, where D-Phe, D-HPhe, D-Tyr, D-HTyr and D-Trp may be substituted by one or more halogens, $(C_1-C_4)$alkyl, $(C_1-C_4)$alkoxy, nitro or trifluoromethyl groups ; D-cyclohexadienyl-Gly ; D-perhydronaphthyl-Ala ; D-perhydrodiphenyl-Ala ; or a basic L- or D-amino acid such as Arg, HArg, Orn, Lys, HLys, Cit, HCit, APhe or ACha, where Arg and HArg may be N-substituted by a $(C_1-C_6)$alkyl or a $(C_3-C_6)$cycloalkyl group on one or both nitrogen atoms, and where Orn, Lys, HLys, APhe and ACha may be N-substituted by one or two $(C_1-C_6)$alkyl or $(C_3-C_6)$cycloalkyl groups, or by an aminotriazolyl or a nicotinoyl, isonicotinoyl, 6-methyl-nicotinoyl, glycyl-nicotinoyl, nicotinyl-azaglycyl, furyl, glycyl-furyl, furyl-azaglycyl, pyrazinyl, pyrazinyl-carbonyl, picolinoyl, 6-methyl-picolinoyl, shikimyl, shikimyl-glycyl, Fmoc or Boc group;
- A7 is a linear, branched or cyclic aliphatic L-amino acid of 3 to 20 carbon atoms such as Ala, Abu, Aib, 3Aib, Val, Nva, Leu, Ile, Tle, Nle, Hol, Npg, CPa, Cpa, Cba, Cha or Ada, which may be N-alpha-substituted by a $(C_1-C_4)$alkyl group optionally substituted by one or several fluorine atoms;
- A8 is a basic L- or D-amino acid such as Arg, HArg, Om, Lys, HLys, Cit, HCit, APhe or ACha, where Arg or HArg may be N-substituted by a $(C_1-C_6)$alkyl or a $(C_3-C_6)$cycloalkyl group on one or both nitrogen atoms, and where Orn, Lys, HLys, APhe or ACha may be N-substituted by one or two $(C_1-C_6)$alkyl or $(C_3-C_6)$cycloalkyl groups, or by an aminotriazolyl or a nicotinoyl, isonicotinoyl, 6-methyl-nicotinoyl, glycyl-nicotinoyl, nicotinyl-azaglycyl, furyl, glycyl-furyl, furyl-azaglycyl, pyrazinyl, pyrazinyl-carbonyl, picolinoyl, 6-methyl-picolinoyl, shikimyl, shikimyl-glycyi, Fmoc or Boc group;
- Z is $GlyNH_2$ ; D-$AlaNH_2$ ; aza$GlyNH_2$ ; or a group $-NHR_2$ where $R_2$ is a $(C_1-C_4)$alkyl which may be substituted by an hydroxy or one or several fluorine atoms; a $(C_3-C_6)$cycloalkyl ; or a heterocyclic radical selected from morpholinyl, pyrrolidinyl and piperidyl;

as well as their pharmaceutically acceptable salts.

In the present description the term "$(C_1-C_4)$alkyl" denotes methyl, ethyl, n-propyl, i-propyl, n-butyl, i-butyl, s-butyl and t-butyl groups.

The term "$(C_1-C_6)$alkyl" denotes methyl, ethyl, n-propyl, i-propyl, n-butyl, i-butyl, s-butyl, t-butyl, n-pentyl, i-pentyl, s-pentyl, t-pentyl and hexyl groups.

The term "$(C_1-C_8)$alkyl" denotes methyl, ethyl, n-propyl, i-propyl, n-butyl, i-butyl, s-butyl, t-butyl, n-pentyl, i-pentyl, s-pentyl, t-pentyl, hexyl, heptyl and octyl groups;

The term "$(C_1-C_4)$alkoxy" denotes a group -OR where R is a $(C_1-C_4)$alkyl.

The term "$(C_2-C_7)$acyl" denotes a group -COR where R is a $(C_1-C_6)$alkyl.

The term "$(C_3-C_6)$cycloalkyl" denotes cyclopropyl, cyclobutyl, cyclopentyl and cyclohexyl groups.

The term "sugar moiety" denotes D- or L-pentoses or hexoses and their amino-derivatives.

The term "LH-RH analogues" denotes peptides in which at least one amino acid has been modified in the sequence of LH-RH.

The term "(S)spirolactam-Pro" denotes the residue of the formula:

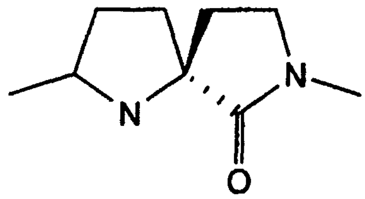

The term "oral administration" denotes the delivery of the peptide analogues of the invention to the gastrointestinal tract by means of an oral formulation or composition.

Peptidomimetic analogues of LH-RH defined by the absence of at least one peptide amide bond, as exemplified in the latest review by Kutscher et al. **(1997,** *Angew. Chem. Int. Ed. Engl.*, **36:** 2148-2161), are not considered within the scope of the present invention.

In the present description and in the claims, the following abbreviations are used:

| | |
|---|---|
| Abu : 2-aminobutyric acid | Ac : acetyl |
| ACha : aminocyclohexylalanine | Aib : 2-aminoisobutyric acid |
| 3Aib : 3-aminoisobutyric acid | Ala : alanine |
| $AlaNH_2$ : alaninamide | APhe : p-aminophenylalanine |
| Arg : arginine | Asp : aspartic acid |
| azaAla : aza-alanine | azaGly : aza-glycine |
| $azaGlyNH_2$ : azaglycinamide | Bal: benzothienylalanine |
| Boc : *tert*-butoxycarbonyl | Cba : cyclobutylalanine |
| Cha : cyclohexylalanine | Cit: citrulline |
| CPa : cyclopropylalanine | Cpa : cylopentylalanine |
| Fmoc : fluorenylmethoxycarbonyl | For : formyl |
| Glu : glutamic acid | Gly : glycine |
| $GlyNH_2$ : glycinamide | HArg: homoarginine |
| HCit : homocitrulline | His : histidine |
| HLys : homolysine | Hot: homoleucine |
| Ile: isoleucine | IprLys : $N^{\varepsilon}$-isopropyllysine |
| Leu : leucine | Lys : lysine |
| MeSer: N-methylserine | Met: methionine |
| Nal : 3-(2-naphtyl)alanine | 1 Nal : 3-(1-naphtyl)alanine |
| NEt: N-ethytamide | NicLys : $N^{\varepsilon}$-nicotinoyllysine |
| Nle : norleucine | Npg : neopentylglycine |
| Nva: norvaline | $OBu^t$ : *tert*-butoxy |
| OBzl: benzyl ester | Orn : omithine |
| Pal : 3-(3-pyridyl)alanine | pClPhe : 3-(4-chlorophenyl)alanine |
| Pen : penicillamine | pGlu : pyroglutamic acid |
| Phe: phenylalanine | Pro : proline |
| Qal : 3-(3-quinolyl)alanine | Sar : sarcosine |
| Ser : serine | (S-Me)Pen : S-methyl-penicillamine |
| (S-Et)Pen : S-ethyl-penicillamine | Thr : threonine |
| Tle : tert-leucine | Trp : tryptophan |
| Tyr : tyrosine | Val : valine |
| Ada : adamantylalanine | HPhe : homophenylalanine |
| MeNpg : N-methylneopentylglycine | 4Pal : 3-(4-pyridyl)alanine |
| HTyr: homotyrosine | 2MeTrp : 2-methyltryptophan |
| Bzl: benzyl | SPL: (S)spirolactam-Pro |
| Asn : asparagine | MeLeu : N-methylleucine |
| MeTyr: N-methyltyrosine | MeHTyr: N-methylhomotyrosine |

A preferred group of peptide analogues (A) comprises the peptides of the formula (SEQ ID N° : 2):

A1-His-A3-A4-A5-A6-A7-A8-Pro-Z (I)

in which:

- A1 is pGlu, Sar or AcSar;
- A3 is an aromatic L-amino acid such as Phe, HPhe, Tyr, HTyr, Trp, 2MeTrp, Nal, 1Nal, diphenyl-Ala, Bal, Pal, 4Pal or Qal, where Phe, HPhe, Tyr, HTyr and Trp may be substituted by one or more halogens, $(C_1-C_4)$alkyl, $(C_1-C_4)$ alkoxy, nitro or trifluoromethyl groups;
- A4 is Ala, Ser, D-Ser, MeSer, Ser($OBu^t$), Ser(OBzl) or Thr;
- A5 is an aromatic L-amino acid such as Phe, HPhe, Tyr, HTyr, Trp, 2MeTrp, Nal, 1 Nal, diphenyl-Ala, Bal, Pal, 4Pal or Qal, where Phe, HPhe, Tyr, HTyr and Trp may be substituted by one or more halogens, $(C_1-C_4)$alkyl, $(C_1-C_4)$ alkoxy, nitro or trifluoromethyl groups;
- A6 is Gly; D-Pro ; (S)-spirolactam-Pro ; D-Ser; D-Thr; D-Cys ; D-Met; D-Pen ; D-(S-Me)Pen ; D-(S-Et)Pen ; D-Ser ($OBu^t$) ; D-Asp($OBu^t$) ; D-Glu($OBu^t$) ; D-Thr($OBu^t$) ; D-Cys($OBu^t$) ; D-Ser($OR_1$) where $R_1$ is a sugar moiety ; an aza-amino acid such as azaGly or azaAla ; D-His which may be substituted on the imidazole ring by a $(C_1-C_6)$alkyl or a benzyl group ; an aliphatic D-amino acid with a $(C_1-C_8)$alkyl or a $(C_3-C_6)$cycloalkyl side chain such as D-Ala, D-Abu, D-Aib, D-3Aib, D-Val, D-Nva, D-Leu, D-Ile, D-Tle, D-Nle, D-Hol, D-Npg, D-CPa, D-Cpa, D-Cba or D-Cha ; an aromatic D-amino acid such as D-Phe, D-HPhe, D-Tyr, D-HTyr, D-Trp, D-2MeTrp, D-Nal, D-1Nal, D-diphenyl-Ala, D-anthryl-Ala, D-phenanthryl-Ala, D-benzhydryl-Ala, D-fluorenyl-Ala, D-Bal, D-Pal, D-4Pal or D-Qal, where D-Phe, DHPhe, D-Tyr, D-HTyr and D-Trp may be substituted by one or more halogens, $(C_1-C_4)$alkyl, $(C_1-C_4)$alkoxy, nitro or trifluoromethyl groups ; D-cyclohexadienyl-Gly ; D-perhydronaphtyl-Ala ; D-perhydrodiphenyl-Ala; or a basic D-amino acid such as D-Arg, D-HArg, D-Orn, D-Lys, D-HLys, D-Cit, D-HCit, D-APhe optionally substituted by an aminotriazolyl group or D-ACha, where D-Arg and D-HArg may be be N-substituted by a $(C_1-C_6)$alkyl or $(C_3-C_6)$ cycloalkyl groups, or by a Fmoc or Boc group;
- A7 is a linear, branched or cyclic aliphatic L-amino acid of 3 to 20 carbon atoms such as Ala, Abu, Aib, 3Aib, Val, Nva, Leu, Ile, Tle, Nle, Hol, Npg, CPa, Cpa, Cba, Cha or Ada, which may be N-alpha-substituted by a $(C_1-C_4)$alkyl group optionally substituted by one or several fluorine atoms;
- A8 is a basic L-amino acid such as Arg, HArg, Om, Lys, HLys, Cit, HCit, APhe optionally substituted by an aminotriazolyl group, or ACha;
- Z is $GlyNH_2$; $azaGlyNH_2$; or a group $-NHR_2$ where $R_2$ is a $(C_1-C_4)$alkyl which may be substituted by an hydroxy or one or several fluorine atoms ; a $(C_3-C_6)$cycloalkyl ; or a heterocyclic radical selected from morpholinyl, pyrrolidinyl and piperidyl;

as well as their pharmaceutically acceptable salts.

Among the peptide analogues of formula (I), those of the formula (SEQ ID N° :3):

pGlu-His-A3-Ser-A5-A6-A7-Arg-Pro-Z (II)

in which:

- A3 and A5 are aromatic L-amino acids as defined for (I) ;
- A6 is as defined for (I) ;
- A7 is Leu, Tie, Nle, Hol, Npg, Cha or Ada, which may be N-alpha-substituted by a methyl or ethyl group optionally substituted by one or several fluorine atoms;
- Z is as defined for (I) ;

as well as their pharmaceutically acceptable salts, are preferred.

Especially preferred are the peptide analogues of the formula (SEQ ID N° ; 4):

pGlu-His-A3-Ser-A5-A6-A7-Arg-Pro-Z (III)

in which:

- A3 and A5 are each independently Phe, Tyr, Trp, 2MeTrp, HPhe, HTyr, Nal, 1Nal, Bal, Pal, 4Pal, or pClPhe;
- A6 is (S)-spirolactam-Pro ; Gly; D-Pro ; D-Ser(OBu$^t$) ; D-Asp(OBu$^t$) ; D-Glu(OBu$^t$) ; D-Thr(OBu$^t$) ; D-Cys(OBu$^t$); D-His or D-His(Bzl) ; D-Ala, D-Leu, D-Tle, D-Nle, D-Hol, D-Npg or D-Cha ; D-Phe, D-HPhe, D-Tyr, D-HTyr, D-Trp, D-2MeTrp, D-Nal, D-1Nal, D-Bal, D-Pal, D-4Pal, or D-pClPhe; D-cyclohexadienyl-Gly; D-perhydronaphtyl-Ala ; D-perhydrodiphenyl-Ala ; or D-APhe optionally substituted by an aminotriazolyl group;
- A7 is Leu, Npg or Cha, which may be N-alpha-substituted by a methyl group;
- Z is $GlyNH_2$; $azaGlyNH_2$ or $-NC_2H_5$.

Also especially preferred are the peptide analogues of the formula (SEQ ID N° : 5) :

pGlu-His-Trp-Ser-Tyr-A6-A7-Arg-Pro-Z (IV)

in which:

- A6 is (S)-spirolactam-Pro, D-Leu, D-Ala, D-Nal, D-Phe, D-Ser(OBu$^t$) or D-Trp;
- A7 is Leu, MeLeu, Npg or MeNpg;
- Z is $GlyNH_2$; $azaGlyNH_2$ or $-NC_2H_5$.

The peptide analogues of formula (I) to (IV) in which A7 is Npg are especially preferred.
Representative peptide analogues of formula (I) to (IV) include leuprorelin, [Npg$^7$]-leuprorelin, triptorelin, [Npg$^7$]-triptorelin, goserelin, [Npg$^7$]-goserelin, buserelin and [Npg$^7$]-buserelin.
Another preferred group of peptide analogues (A) comprises the peptides of the formula (SEQ ID N°: 6):

A1-A2-A3-A4-A5-A6-A7-A8-Pro-Z (I')

in which:

- A1 is pGlu ; D-pGlu ; Sar; AcSar ; Pro or a derivative thereof such as AcPro, ForPro, OH-Pro, Ac-OH-Pro, dehydro-Pro or Ac-dehydro-Pro ; Ser; D-Ser ; Ac-D-Ser ; Thr ; D-Thr ; Ac-D-Thr ; or an aromatic D-amino acid which may be acylated, preferably acetylated, such as D-Phe, D-HPhe, D-Tyr, D-Trp, D-Nal, D-1Nal, D-diphenyl-Ala, D-Bal, D-Pal, D-4Pal or D-Qal, where D-Phe and D-Trp may be substituted by one or more halogens, $(C_1$-$C_4)$alkyl, $(C_1$-$C_4)$ alkoxy, nitro or trifluoromethyl groups;
- A2 is a direct bond or an aromatic D-amino acid such as D-Phe, D-HPhe, D-Tyr, D-Trp, D-Nal, D-1Nal, D-diphenyl-Ala, D-Bal, D-Pal, D-4Pal or D-Qal, where D-Phe and D-Trp may be substituted by one or more halogens, $(C_1$-$C_4)$ alkyl, $(C_1$-$C_4)$alkoxy, nitro or trifluoromethyl groups;
- A3 is an aromatic L- or D-amino acid such as Phe, HPhe, Tyr, Trp, Nal, 1Nal, diphenyl-Ala, Bal, Pal, 4Pal or Qal, where Phe and Trp may be substituted by one or more halogens, $(C_1$-$C_4)$alkyl, $(C_1$-$C_4)$alkoxy, nitro or trifluoromethyl groups;
- A4 is Ala, Ser, D-Ser, MeSer, Ser(OBu$^t$), Ser(OBzl) or Thr;
- A5 is an aromatic L-amino acid such as Phe, HPhe, Tyr, HTyr, Trp, Nal, 1Nal, diphenyl-Ala, Bal, Pal, 4Pal or Qal, where Phe, Tyr, HTyr and Trp may be substituted by one or more halogens, $(C_1$-$C_4)$alkyl, $(C_1$-$C_4)$alkoxy, nitro or trifluoromethyl groups and/or N-alpha-substituted by a $(C_1$-$C_4)$alkyl group optionally substituted by one or several fluorine atoms; or a basic L- or D-amino acid such as Arg, HArg, Orn, Lys, HLys, Cit, HCit, APhe or ACha, where Arg and HArg may be N-substituted by a $(C_1$-$C_6)$alkyl or $(C_3$-$C_6)$cycloalkyl group on one or both nitrogen atoms, and where Orn, Lys, HLys, APhe and ACha may be N-substituted by one or two $(C_1$-$C_6)$alkyl or $(C_3$-$C_6)$cycloalkyl groups, or by a nicotinoyl, isonicotinoyl, 6-methyl-nicotinoyl, glycyl-nicotinoyl, nicotinyl-azaglycyl, furyl, glycyl-furyl, furyl-azaglycyl, pyrazinyl, pyrazinyl-carbonyl, picolinoyl, 6-methyl-picolinoyl, shikimyl, shikimyl-glycyl, Fmoc or Boc group;
- A6 is Gly; (S)-spirolactam-Pro ; D-Pro ; D-Ser ; D-Thr ; D-Cys ; D-Met ; D-Asn ; D-Pen ; D-(S-Me)Pen ; D-(S-Et) Pen ; D-Ser(OBu$^t$) ; D-Asp(OBu$^t$) ; D-Glu(O-Bu$^t$) ; D-Thr(O-Bu$^t$) ; D-Cys(O-Bu$^t$) ; D-Ser(O-$R_1$) where $R_1$ is a sugar moiety ; an aliphatic D-amino acid with a $(C_1$-$C_8)$alkyl or a $(C_3$-$C_6)$cycloalkyl side chain such as D-Ala, D-Abu, D-Aib, D-3Aib, D-Val, D-Nva, D-Leu, D-Ile, D-Tle, D-Nle, D-Hol, D-Npg, D-CPa, D-Cpa, D-Cba or D-Cha ; an aromatic D-amino acid such as D-Phe, D-HPhe, D-Tyr, D-Trp, D-Nal, D-1Nal, D-diphenyl-Ala, D-anthryl-Ala, D-phenanthryl-Ala, D-benzhydryl-Ala, D-fluorenyl-Ala, D-Bal, D-Pal, D-4Pal or D-Qal, where D-Phe and D-Trp may be substituted by one or more halogens, $(C_1$-$C_4)$alkyl, $(C_1$-$C_4)$alkoxy, nitro or trifluoromethyl groups ; D-cyclohexadienyl-Gly ; D-perhydronaphthyl-Ala ; D-perhydrodiphenyl-Ala; or a basic L- or D-amino acid such as Arg, HArg,

Orn, Lys, HLys, Cit, HCit, APhe or ACha, where Arg and HArg may be N-substituted by a ($C_1$-$C_6$)alkyl or ($C_3$-$C_6$) cycloalkyl group on one or both nitrogen atoms, and where Orn, Lys, HLys, APhe and ACha may be N-substituted by one or two ($C_1$-$C_6$)alkyl or ($C_3$-$C_6$)cycloalkyl groups, or by a nicotinoyl, isonicotinoyl, 6-methyl-nicotinoyl, glycyl-nicotinoyl, nicotinyl-azaglycyl, furyl, glycyl-furyl, furyl-azaglycyl, pyrazinyl, pyrazinyl-carbonyl, picolinoyl, 6-methyl-picolinoyl, shikimyl, shikimyl-glycyl, Fmoc or Boc group;

- A7 is a linear, branched or cyclic aliphatic L-amino acid of 3 to 20 carbon atoms such as Ala, Abu, Aib, 3Aib, Val, Nva, Leu, Ile, Tle, Nle, Hol, Npg, CPa, Cpa, Cba, Cha or Ada, which may be N-alpha-substituted by a ($C_1$-$C_4$)alkyl group optionally substituted by one or several fluorine atoms ;
- A8 is a basic L- or D-amino acid such as Arg, HArg, Orn, Lys, HLys, Cit, HCit, APhe or ACha, where Arg and HArg may be N-substituted by a ($C_1$-$C_6$)alkyl or ($C_3$-$C_6$)cycloalkyl group on one or both nitrogen atoms, and where Om, Lys, HLys, APhe and ACha may be N-substituted by one or two ($C_1$-$C_6$)alkyl or ($C_3$-$C_6$)cycloalkyl groups, or by a nicotinoyl, isonicotinoyl, 6-methyl-nicotinoyl, glycyl-nicotinoyl, nicotinyl-azaglycyl, furyl, glycyl-furyl, furyl-azaglycyl, pyrazinyl, pyrazinyl-carbonyl, picolinoyl, 6-methyl-picolinoyl, shikimyl, shikimyl-glycyl, Fmoc or Boc group;
- Z is $GlyNH_2$ or $D\text{-}AlaNH_2$;

as well as their pharmaceutically acceptable salts.

Among the peptides of formula (I'), those of the formula (SEQ ID N° : 7):

$$\text{Ac-D-Nal-D-pClPhe-D-Pal-Ser-A5-A6-A7-A8-Pro-D-AlaNH}_2 \qquad \text{(II')}$$

in which:

- A5 is Tyr, HTyr, MeTyr, MeHTyr, NicLys or IprLys;
- A6 is (S)-spirolactam-Pro, D-Arg, D-NicLys, D-IprLys, D-Cit, D-HCit or D-Asn;
- A7 is Leu, MeLeu, Npg or MeNpg;
- A8 is Arg, NicLys or IprLys;
  and their pharmaceutically acceptable salts,
  are preferred.

The peptide analogues of formula (I') and (II') in which A7 is Npg are especially preferred.

Representative peptide analogues of formula (I') and (II') include antide, [$Npg^7$]-antide, cetrorelix, [$Npg^7$]-cetrorelix, abarelix and [$Npg^7$]-abarelix.

Further preferred peptide analogues comprise those of formula (A) where A6 is as defined therein except D-Asn.

Examples of the salts with pharmaceutically acceptable acids are those with mineral acids, such as for example the hydrochloride, hydrobromide, sulfate, phosphate, borate, hydrogensulfate, dihydrogenphosphate or nitrate, and those with organic acids, such as for example the acetate, oxalate, tartrate, succinate, maleate, fumarate, gluconate, citrate, pamoate, malate, ascorbate, benzoate, p-toluenesulfonate or naphtalenesulfonate.

Examples of the salts with pharmaceutically acceptable bases are those with alkali or alkaline earth metals such as sodium, potassium, calcium or magnesium, and those with organic bases such as amines, trometamol, N-methylglutamine, and the like.

The peptides used in the present invention can be prepared by the well-known techniques of peptide chemistry such as for example peptide synthesis in solution or solid phase peptide synthesis. In general, these techniques involve the stepwise addition of one or more amino acids -which may be suitably protected- to a forming peptide chain. Reference can for example be made to *Synthetic Peptides: a user's guide,* ed. by G.A. Grant, 1992, UWBC Biotechnical Resource Series, Washington University Press, Saint-Louis, USA.

Each molecule of α-CD bears 6 primary hydroxyl groups and 12 secondary hydroxyl groups, respectively correspondind to the 6-OH and to the 2- and 3-OH groups of each of the 6 glucopyranose units. Another general aspect of the present invention concerns α-CD and its derivatives which are defined as the result of chemical or biochemical modifications involving a precise or average number between 1 and 18 hydroxyl groups of the α-CD molecule, in a random or regioselective fashion, with one or several different types of reactions such as oxidation, reduction, alkylation, hydroxyalkylation, esterification with organic or mineral acids, intramolecular dehydration, tosylation followed by reductive amination or halogen substitution, sugar branching or further polymerization, and their different possible combinations and mixtures. Examples of α-CD derivatives include α-CD modified with one or more groups selected from methyl, carboxymethyl, ethyl, butyl, octyl, hydroxyethyl, hydroxypropyl, hydroxybutyl, acetyl, propionyl, butyryl, succinyl, benzoyl, palmityl, sulfonyl, toluenesulfonyl, amino, aminopropyl, glucosyl, maltosyl, dimaltosyl, carboxymethyl

ether, sulfobutylether, and phosphate ester.

Preferred α-CD derivatives according to the invention comprise methylated α-CD ; hexakis (2,3,6-tri-O-methyl)-α-CD, also known as "permethylated" α-CD ; carboxymethylated α-CD and phosphated α-CD. α-CD and hexakis (2,3,6-tri-O-methyl)-α-CD are especially advantageous when used in the preparation of the pharmaceutical compositions of the invention.

As mentioned above, α-CD or its derivatives enhance the biological activity of LH-RH peptide analogues in oral pharmaceutical compositions.

Thus, according to another feature, the invention relates to oral pharmaceutical compositions which comprise as the active principle a LH-RH peptide analogue as defined above in the form of a combination with α-CD or a derivative thereof, said compositions being intended to be delivered to the gastrointestinal tract.

The peptides according to the general formula (I) exert an agonist activity upon the LH-RH receptors *in vivo*, resulting in the stimulation of LH secretion by the pituitary, which, in males, stimulates the secretion of testosterone by the testis.

Adult male Sprague-Dawley rats were orally administered by gavage an oral formulation comprising leuprorelin (LEU, Bachem), triptorelin (TRI, Bachem), deslorelin (DES, Saxon Biochemicals), goserelin (GOS, Saxon Biochemicals) or the other following example analogues: example 1 ([(S)spirolactam($Pro^6$, $Npg^7$), $desGly^{10}$-$ProNEt^9$]LH-RH), example 2 ([D-$Ala^6$, Npg7, $desGly^{10}$-$ProNEt^9$]LH-RH), example 3 ([$Npg^7$]leuprorelin), example 4 ([D-$Phe^6$, Npg7, $desGly^{10}$-$ProNEt^9$]LH-RH), example 5 ([$Npg^7$]triptorelin) and example 6 ([D-$Ala^6$, $desGly^{10}$-$ProNet^9$]LH-RH, Bachem), in combination with α-CD (Sigma or Wacker Chemie). As a comparison, the same agonists have been orally administered by gavage in a standard aqueous vehicle not comprising α-CD (comparative examples). For screening purposes, blood samples were drawn 2 hours after oral administration of a common dose of 2 nmoles/rat of LH-RH peptide agonists in an aqueous solution containing 10 or 100 mM of α-CD (Tables 1 and 2; Figures 1, 4 and 6). For kinetic purposes, the effects of 2 nmoles/rat of example 3 with or without 100 mM of α-CD were tested between 0.5 and 8 hours on plasma LH and testosterone levels (Figures 2 and 5). The influence of increasing concentrations of α-CD (5%, 10% or 14%) was tested with example 2 at the dose of 5 Ng/kg 2 hours after oral administration (Figure 3). Total plasma testosterone (Diagnostic System Laboratories) and LH (Amersham Pharmacia Biotech) determinations were performed by radioimmunoassay. In screening 2-hour experiments, each group comprised between 6 and 8 rats; each time point of the kinetic study was studied on four animals.

Table 1 :

| stimulation of testosterone secretion | | | |
|---|---|---|---|
| Compound without α-CD | total plasma testosterone (nmol/l) (m ± sem) | Compound with α-CD | total plasma testosterone (nmol/l) (m ± sem) |
| Control | 8.6 ± 3.32 | Control (α-CD) | 3.8 ± 0.66 |
| Triptorelin | 25.8 ± 3.14 | Triptorelin (α-CD) | 61.9 ± 6.01 |
| Leuprorelin | 26.3 ± 5.77 | Leuprorelin (α-CD) | 70.7 ± 4.06 |
| Goserelin | 21.4 ± 5.99 | Goserelin (α-CD) | 66.5 ± 6.19 |
| Deslorelin | 9.7 ± 2.41 | Deslorelin (α-CD) | 48.2 ± 7.29 |
| C. ex 1 | 40.1 ± 6.78 | Example 1 | 58.0 ± 8.75 |
| C. ex 2 | 23.0 ± 8.54 | Example 2 | 72.8 ± 4.64 |
| C. ex 3 | 39.7 ± 8.11 | Example 3 | 69.7 ± 3.6 |
| C. ex 4 | 30.1 ±5.86 | Example 4 | 67.9 ± 9.11 |
| C. ex 5 | 15.8 ± 4.24 | Example 5 | 52.1 ± 6.99 |
| C. ex 6 | 28.3 ± 4.56 | Example 6 | 61.8 ± 5.10 |

As can be seen from the above results as well as from Figures 1-4, oral formulations with α-CD significantly enhance the stimulation of testosterone secretion induced by LH-RH analogues. Especially, deslorelin alone was inactive at this threshold dose of 2 nmoles/rat, but showed a marked potency when formulated with α-CD. The crucial role played by α-CD is demonstrated by the concentration-dependance of its effect: combined with 10 mM α-CD (0.972%), the oral activity of example 3 was not significantly improved (Figure 1); at 5% (51.4 mM), α-CD did enhance the stimulation of testosterone secretion induced by example 2 by oral administration, although not to the maximal level achieved with 10% (103 mM) as well as 14% (144 mM) (Figure 3).

It is also worth noting (see figures 1 and 3) that β-CD, hydroxypropyl-β-CD (HP-β-CD) and γ-CD have no potentiating effect on the LH-RH analogue-induced stimulation of testosterone secretion.

Table 2:

| stimulation of LH secretion | | | |
|---|---|---|---|
| Compound without α-CD | total plasma LH (ng/ml) (m ± sem) | Compound with α-CD | total plasma LH (ng/ml) (m ± sem) |
| Control | 1.2 ± 0.11 | Control (α-CD) | 1.1 ± 0.10 |
| Triptorelin | 1.4 ± 0.10 | Triptorelin (α-CD) | 10.1 ± 2.54 |
| Leuprorelin | 1.2 ± 0.14 | Leuprorelin (α-CD) | 12.3 ± 2.03 |
| C. ex 1 | 1.5 ± 0.19 | Example 1 | 7.1 ± 1.68 |
| C. ex 2 | 1.6 ± 0.14 | Example 2 | 19.7 ± 3.70 |
| C. ex 3 | 2.2 ± 0.58 | Example 3 | 10.9 ± 1.66 |
| C. ex 4 | 1.4 ± 0.17 | Example 4 | 16.1 ± 5.22 |
| C. ex 5 | 1.4 ± 0.10 | Example 5 | 3.2 ± 0.56 |

As can be seen from the above results as well as from Figures 5-6, the potentiating effect of α-CD in oral formulations containing LH-RH analogues on LH release is even more pronounced than on testosterone secretion : all tested LH-RH analogues were inactive when administered alone at the same dose of 2 nmoles/rat, whereas, depending on the analogue, they induced a 3- to 16-fold increase above control levels when administered in combination with α-CD.

Similar or even better results were obtained with α-CD derivatives such as methylated α-CD, hexakis(2,3,6-tri-O-methyl)-α-CD, carboxymethylated α-CD or phosphated α-CD. All α-CD derivatives were purchased from Cyclolab (Budapest, Hungary). The influence of α-CD derivatives were compared with that of α-CD itself on the potentiation of LH-RH agonist activity of example 3 when administered by gavage to rats at the low dose of 5 µg/kg p.o. : total testosterone plasma levels were measured 2 hours after administration (Table 3).

Table 3:

| Stimulation of testosterone secretion by example 3 | | | |
|---|---|---|---|
| dose (µg/kg p.o.) | cyclodextrin (CD) type (concentration) | testosterone levels (ng/ml); (m ± sem) | n rats |
| 0 (control) | none | 1.0 ± 0.17 | 24 |
| 5 | none | 3.4 ± 0.79 | 16 |
| 5 | carboxymethylated α-CD (50%) | 6.9 ± 1.62 | 10 |
| 5 | methylated α-CD (30%) | 7.1 ± 1.59 | 10 |
| 5 | phosphated α-CD (30%) | 7.4 ± 2.00 | 10 |
| 5 | α-CD (10%) | 10.0 ± 1.22 | 24 |
| 5 | permethylated α-CD (15%) | 12.9 ± 1.10 | 10 |

The α-CD derivatives tested above at least doubled the effect of example 3 by oral administration. Native α-CD and its permethylated derivative appeared to be especially favorable with respectively a 2.9- and 3.8- fold enhancement of agonist activity of example 3 on testosterone at this dose level of 5 µg/kg p.o.

In a further experiment, example 3 was tested with α-CD or permethylated α-CD at an equal concentration of 10%. Two hours after administration, plasma LH levels were measured on eight rats per dose group (Figure 7). The 5 µg/kg p.o dose of example 3 alone was inactive on LH levels at this time point, and 10 and 20 µg/kg p.o. were clearly threshold doses in these experimental conditions.

Combination of example 3 with 10% α-CD resulted in slight but significant stimulations at 5 and 10 µg/kg p.o., and in a much greater effect at 20 µg/kg p.o. when compared with example 3 alone (over 5-fold enhancement of LH-releasing activity). Moreover, combination of example 3 with 10% permethylated α-CD resulted in an even higher potentiation : the doses of 2.5, 5 and 10 µg/kg p.o., which remained inactive when example 3 was given alone, yielded a sharp dose-related stimulatory response (Figure 7).

The peptides according to the general formula (I') exert an antagonist activity upon the LH-RH receptors *in*

*vivo,* resulting in particular in the inhibition of ovulation.

The influence of α-, β- and γ-CD was tested on the activity of antide (an example of LH-RH peptide antagonist) when orally admininisted by gavage to normally cycling adult female Wistar rats between 1:30 and 3:00 p.m. on the day of proestrus, after at least two full regular estrous cycles as monitored by daily vaginal smears. The antiovulatory efficacy was checked the next morning, on the day of expected estrus, by looking for ova in the oviduct of treated females. The presence of at least one ovum attested that some degree of spontaneous ovulation did occur, and only the total absence of ovum was considered as effective LH-RH antagonist-induced inhibition of ovulation. Antide was solubilized in a vehicle consisting of 20% (vol/vol) propylene glycol in water already containing 1% bovine albumin, to which 10% (wt/vol) of either α-, β- or γ-CD was then added. The results of the experiments are summarized in the following Table 4.

Table 4:

| Inhibition of ovulation by oral administration of antide | | | |
|---|---|---|---|
| oral formulation | antide (µg/rat p.o.) | n ovulations/ N treated rats | percentage of inhibition |
| vehicle only | 0 | 24/24 | 0% |
| vehicle + α-CD (10%) | 0 | 8/8 | 0% |
| vehicle only | 200 | 8/8 | 0% |
| | 400 | 19/22 | 14% |
| | 600 | 5/8 | 44% |
| vehicle + α-CD (10%) | 200 | 6/8 | 25% |
| | 400 | 6/22 | 73% |
| | 600 | 2/8 | 75% |
| vehicle + β-CD (10%) | 400 | 7/7 | 0% |
| vehicle + γ-CD (10%) | 400 | 7/7 | 0% |

The vehicle with or without α-CD had no effect by itself. The threshold effective dose of antide by oral administration was 400 µg/kg p.o. with only 3 animals out of 22 showing inhibition of ovulation. Beta- and γ-CD had no influence on the minimal activity of antide at this dose level.

However, α-CD significantly enhanced antide potency from 14% to 73% of inhibition at 400µpg/kg. The twice lower dose of 200 pg/kg was even slightly effective (25% of inhibition) in combination with α-CD 10%. Therefore, α-CD was able to potentiate the activity of a LH-RH peptide antagonist by oral administration, but not β- or γ-CD.

The oral formulations of the invention can be prepared by methods well known to those skilled in the art, generally as follows : a known amount of a drug is added to an aqueous cyclodextrin solution in sufficient concentration; the drug-cyclodextrin interaction can take place in solution or suspension within minutes or after stirring for up to 1 week at the desired temperature with or without sonication, depending on the nature of the drug and of the cyclodextrin, and on their respective concentrations. Then, the resulting drug-cyclodextrin combination or complex can be further obtained in a dry form by filtration, centrifugation, evaporation or sublimation.

By way of illustration, the example combinations of LH-RH analogues with α-CD hereafter describe one basic method for the preparation of the formulations according to the invention in solution, notwithstanding their further processing to any appropriate dry form that will take advantage of the same potentiating properties.

Such formulations may further comprise, one or several other pharmaceutically appropriate excipients for oral administration such as lactose, fructose, glucose, sucrose, compressible sugar, saccharin, povidone, crospovidone, magnesium stearate, kaolin, bentonite, colloidal silica, mannitol, sorbitol, starch and its derivatives, microcrystalline or powdered cellulose, methylcellulose, carboxymethylcellulose, ethylcellulose or other chemically modified celluloses, other cyclodextrins, maltodextrin, dextrates, dextrin, dextrose, alginates, pectins, pectates, sorbitan esters, polysorbate 80, chitosan, guar or xanthan gums, mono-, di- or tri-ethanolamine, oleic acid or ethyl oleate, stearic acid, water, liquid glucose, propylene glycol, lactic acid, malic acid, ethanol, isopropyl myristate or palmitate, glycerin, glyceryl monooleate, glyceryl monostearate, glyceryl palmitostearate, lecithin, medium or short chain triglycerides, various oils from corn, cottonseed, olive, peanut, sesame or soybean, and the like. These formulations are administered by mouth (or naso-gastric tubing) in various aqueous or non-aqueous solutions or suspensions such as true solutions, syrups, elixirs, mucilages, jellies, gels, milks, magmas, macro-, micro-or nano-emulsions, or in various solid forms such as compressed, coated, buccal, sublingual, effervescent or molded tablets, hard or soft capsules, pills, troches or cachets.

Enteric coatings of usual solid oral dosage forms or of soft capsules containing liquid formulations, and sustained, delayed or programmed gastric, enteric or colonic release forms or devices are preferred means to deliver the active principle.

The main target of LH-RH peptide agonists according to formula (I) is the pituitary gland, but direct actions have been reported on the gonads themselves (testis and ovary), on the thymus and some lymphoid cell lines, and on breast, prostate, pancreatic or nervous system tumors. They exert on any LH-RH sensitive target, either a stimulatory activity by short-term acute or pulsatile administration, or an inhibitory effect by repeated or continuous administrations that induce the desensitization and the down-regulation of LH-RH receptors. In the case of the hypothalamo-pituitary-gonadal axis, prolonged administration results in a so-called "chemical" castration.

The main target of LH-RH peptide antagonists according to formula (I') is also the pituitary gland, where they bind to the LH-RH receptors and prevent the activity of endogenous LH-RH. By this mechanism, the pituitary-gonadal axis can be inhibited. The programmed use of LH-RH antagonists can also be taken advantage of to obtain a spontaneous stimulation of the pituitary-gonadal axis at any given time by stopping their administration at an appropriate earlier time point.

Therefore, LH-RH agonists or antagonists according to formula (A) are useful in all situations where the actions of LH-RH must be either inhibited, prevented or stimulated. Especially, the peptide analogues of the invention are useful in the treatment of LH-RH-sensitive diseases, namely the diseases where a LH-RH agonist or antagonist action is required.

Accordingly, the oral pharmaceutical compositions of the invention can find an appropriate therapeutic use in humans as well as in animals, depending on doses and treatment regimens, in reproductive endocrinology and in the treatment or prevention of sex hormone-dependent benign or malignant tumors ; said treatment or prevention may involve parallel and/or sequential supplementary curative or preventive regimens based on other hormonal or antitumoral agents. LH-RH sensitive sex hormone-independent benign or malignant tumors can also regress upon treatment with the oral pharmaceutical compositions according to the invention, alone or associated with other parallel and/or sequential antitumoral treatments. Immune mechanisms can also be modified by the oral pharmaceutical compositions according to the invention, alone or associated with other parallel and/or sequential treatments based on immuno-modulating or -suppresive agents such as glucocorticoids, cyclosporin, rapamycin, tacrolimus, their derivatives, and the like. The oral pharmaceutical compositions according to the invention are therefore very valuable in the treatment and prevention of autoimmune diseases, graft rejection or atopic diseases, and in the treatment of benign or malignant lymphoproliferative disorders.

The oral pharmaceutical compositions according to the invention are especially useful in the inhibition, planning and triggering of ovulation in *in vitro* fertilization programs, and in the treatment of male and female infertility or hypogonadic states. Conversely, they can also be used in male or female contraception or treatment of hypergonadic states. In both cases, said treatments may involve other parallel and/or sequential treatments with sex steroids and/or gonadotrophins. This applies to men and women, but also to wild or domestic animals in uses such as improvement or control of reproductive performance, or as a tool to optimize breeding strategies.

The oral pharmaceutical compositions according to the invention are also especially useful in men to treat advanced prostate cancer, but can also be used as a first line therapy in this indication and in benign prostatic hypertrophy ; in both cases, said treatments may also involve additional parallel and/or sequential treatments based on inhibitors of androgen action, i.e. antiandrogens such as cyproterone acetate, osaterone acetate, chlormadinone acetate, flutamide, nilutamide or bicalutamide and the like, and/or on 5$\alpha$-reductase inhibitors such as finasteride, epristeride or turosteride and the like, and/or on $C_{17-20}$ lyase inhibitors such as abiraterone and the like.

The oral pharmaceutical compositions according to the invention are also especially useful in the treatment or prevention of breast cancer in women and in men, especially estrogen receptor positive tumors ; said treatment or prevention may involve parallel or sequential supplementary curative or preventive regimens based on antiestrogens such as tamoxifen, raloxifen or droloxifen and the like, and/or on aromatase inhibitors such as atamestane, formestane, letrozole, anastrozole and the like, and/or on $C_{17-20}$ lyase inhibitors such as abiraterone and the like. The oral pharmaceutical compositions according to the invention are also very useful in the treatment or prevention of certain estrogen receptor negative tumors that respond to the direct effects of LH-RH analogues or indirectly to their gonadal suppressive activity.

Other gynecological conditions, such as endometrial hyperplasia, leiomyoma, adenomyoma, endometriosis, polycystic ovary syndrome, hirsutism and benign breast disease (pain, cysts or fibrosis), can also be prevented by or benefit from treatment with the oral pharmaceutical compositions according to the invention ; said treatment or prevention may involve additional parallel and/or sequential curative or preventive treatments based on antiestrogens (cited above), progestins such as cyproterone acetate, osaterone acetate, chlormadinone acetate, nomegestrol acetate, promegestone, demegestone, trimegestone and the like, and/or their contraceptive or post-menopausal replacement combination formulations with estrogens such as estradiol or ethynylestradiol. The oral compositions of the invention can also interfere with gestation by inducing abortion or by triggering labor; in this case they may also be used

in parallel or in sequence with treatments based on estrogens (cited above), antiprogestins such as mifepristone and/or prostaglandin analogs such as sulprostone.

Similar indications can be encountered in veterinary medicine for male or female domestic or wild animals that may require the use of pharmaceutical compositions according to the invention.

A further aspect of the invention relates to the use of a composition comprising an effective amount of a LH-RH peptide analogue as previously defined in combination with α-cyclodextrin or a derivative thereof for the manufacture of a pharmaceutical composition according to the invention for treating and/or preventing the above diseases by orally administering said pharmaceutical composition to patients or animals in need thereof. Said use may comprise the further administration of at least one of the active principles mentioned above such as for example a hormonal agent, an antitumoral agent, an immuno-modulating or suppressive agent, a sex steroid, a gonadotrophin, an inhibitor of androgen action, a 5α-reductase inhibitor, a $C_{17-20}$ lyase inhibitor, an antiestrogen, an aromatase inhibitor, a progestin, an estrogen, an antiprogestin or a prostaglandin analogue, said further administration being parallel, sequential or over a period of time.

The unit dose of oral administration of LH-RH peptide analogues according to formula (A) may range from 0.1 to 100 mg per human patient, from one to 16 times per day (in the case of pulsatile administration), in combination with at least an equimolar amount of α-CD or its derivatives and up to the total remaining part of the oral formulation.

All the above-mentioned oral pharmaceutical compositions may additionally contain one or several proteases inhibitors, and/or one or several other absorption enhancers.

<u>Examples of preparations of leuprorelin, triptorelin, goserelin, deslorelin and examples 1 to 6 in combination with 100 mM α-CD in solution</u>

On each experimental day, solutions of α-CD were freshly prepared by dissolving 9.72 g in 100 ml of pure water, or 4.86 g in 50 ml, for 1 hour at room temperature with gentle magnetic stirring; meanwhile, an appropriate volume of each LH-RH analogue was taken from thawed individual stock vials containing 50 µg of net peptide in 50 µl of phosphate-buffered saline containing 0.1% bovine serum albumin, to make 20 nmoles (24.2 µl for LEU, 26.2 µl for TRI, 23.4 µl for GOS, 25.4 µl for DES, 24.7 µl for example 1, 23.6 µl for example 2, 24.5 µl for example 3, 25.1 µl for example 4, 26.5 µl for example 5 and 23.4 µl for example 6) and put in a 10 ml gauged flask. Then, the α-CD solution was added to fill the flask up to 10 ml to make a 2 nmol/ml solution of which 1ml was administered by oral gavage to each rat.

<u>Examples of preparation of formulations of example 2 in combination with 5%, 10% or 14% α-CD solutions</u>

On each experimental day, 45 µl of one thawed individual vial containing 50 µg of net example 2 in 50 µl of phosphate-buffered saline containing 0.1 % bovine serum albumin, were diluted in 36 ml of distilled water to give a 1.25 µg/ml solution from which three fractions of 3.8 ml were taken; then, 190, 380 or 532 mg of α-CD were added to each fraction to give a concentration of 5%, 10% or 14%, respectively. After overnight magnetic stirring at room temperature, each solution was given to rats by oral gavage in a 4 ml/kg volume to administer the same dose of 5 µg/kg of example 2 without or with increasing concentrations of α-CD.

<u>Examples of preparation of formulations for oral administration of example 3 in combination with α-CD derivatives</u>

On each experimental day, frozen vials containing 50 µg of net example 3 in 50 µl of phosphate-buffered saline containing 0.1% bovine serum albumin were thawed and diluted by half with an equal volume of the same fresh bovine serum albumin solution. Then, 12.5 µl of this 0.5 µg/µl solution were added to 5 ml of aqueous vehicle for oral administration (with or without α-CD derivative) to give a final formulation containing 1.25 µg/ml of example 3 to be administered by gavage under a volume of 4 ml/kg, after gentle magnetic stirring overnight.

The solutions of α-CD derivatives were prepared by weighing the appropriate amount to put in 10 ml gauged flasks to fill up with water: 5 g of carboxymethylated α-CD (50%), 3 g of methylated α-CD (30%), 3 g of phosphated α-CD (30%) or 1 or 1.5 g of permethylated α-CD (10 or 15%).

Appropriate volumes of convenient dilutions of the 50 µg/50 µl stock vials of net example 3 were added to 10% solutions of α-CD or permethylated α-CD in water to obtain the dose range described in figure 7.

<u>Examples of preparations of formulations for oral administration of antide in combination with α-, β- or γ-CD</u>

Each 5 mg powder vial of antide (from Bachem, Bubendorf, Switzerland) containing 4.2434 mg net peptide was dissolved in a mixture of 2.122 ml propylene glycol with 8.487 ml water containing 0.1% bovine albumin. To each 10.609 ml solution of antide (400 µg/ml), 1.061 g of either α-, β- or γ-CD was directly added to obtain a 10% concentration. Each female rat received the same volume of 1 ml of test formulation by gavage.

The same volume of administration was used for the doses of 200 and 600 μg/rat. The appropriate concentrations of antide to be administered (200 and 600 μg/ml) were respectively obtained by diluting by half the 400 μg/ml solution with the same [20% vol. propylene glycol/80% vol. albuminated water/10% wt α-CD] mixture, and by dissolving other 5 mg powder vials in 1.415 ml propylene glycol with 5.658 ml water already containing 0.1% bovine albumin to which 0.707 mg of α-CD was finally added.

## Claims

**1.** Use of α-cyclodextrin or a derivative thereof for the preparation of a pharmaceutical composition for the oral administration of a LH-RH peptide analogue or one of its pharmaceutically acceptable salt.

**2.** Use according to claim 1 wherein said peptide analogue has the formula (SEQ ID N° : 1) :

A1-A2-A3-A4-A5-A6-A7-A8-Pro-Z (A)

in which:

- A1 is pGlu ; D-pGlu ; Sar; AcSar; Pro or a derivative thereof; Ser; D-Ser ; Ac-D-Ser; Thr; D-Thr ; Ac-D-Thr ; or an aromatic D-amino add which may be acylated;
- A2 is a direct bond; His; or an aromatic D-amino acid;
- A3 is an aromatic L- or D-amino acid;
- A4 is Ala, Ser, D-Ser, MeSer, Ser($OBu^t$), Ser(OBzl) or Thr;
- A5 is an aromatic L-amino acid or a basic L- or D-amino acid ;
- A6 is Gly; (S)-spirolactam-Pro ; D-Pro ; D-Ser ; D-Thr ; D-Cys ; D-Met ; D-Asn ; D-Pen ; D-(S-Me)Pen ; D-(S-Et)Pen ; D-Ser($OBu^t$) ; D-Asp($OBu^t$) ; D-Glu($OBu^t$) ; D-Thr($OBu^t$) ; D-Cys($OBu^t$) ; D-Ser($OR_1$) where $R_1$ is a sugar moiety ; an aza-amino acid ; D-His which may be substituted on the imidazole ring by a ($C_1$-$C_6$) alkyl, a ($C_2$-$C_7$)acyl or a benzyl group ; an aliphatic D-amino acid with a ($C_1$-$C_8$)alkyl or a ($C_3$-$C_6$)cycloalkyl side chain ; an aromatic D-amino add ; D-cydohexadienyl-Gly ; D-perhydronaphthyl-Ala; D-perhydrodiphenyl-Ala; or a basic L- or D-amino acid;
- A7 is a linear, branched or cyclic aliphatic L-amino acid of 3 to 20 carbon atoms which may be N-alpha-substituted by a ($C_1$-$C_4$)alkyl group optionally substituted by one or several fluorine atoms;
- A8 is a basic L- or D-amino acid;
- Z is $GlyNH_2$; D-$AlaNH_2$ ; aza$GlyNH_2$ ; or a group -$NHR_2$ where $R_2$ is a ($C_1$-$C_4$)alkyl which may be substituted by an hydroxy or one or several fluorine atoms ; a ($C_3$-$C_6$)cycloalkyl ; or a heterocyclic radical selected from morpholinyl, pyrrolidinyl and piperidyl.

**3.** Use according to claim 2 wherein said peptide analogue has the formula (SEQ ID N°: 2):

A1-His-A3-A4-A5-A6-A7-A8-Pro-Z (I)

in which:

- A1 is pGlu, Sar or AcSar;
- A3 is an aromatic L-amino acid;
- A4 is Ala, Ser, D-Ser, MeSer, Ser($OBu^t$), Ser(OBzl) or Thr;
- A5 is an aromatic L-amino acid ;
- A6 is Gly; D-Pro ; (S)-spirolactam-Pro ; D-Ser; D-Thr; D-Cys ; D-Met; D-Pen ; D-(S-Me)Pen; D-(S-Et)Pen ; D-Ser($OBu^t$) ; D-Asp($OBu^t$); D-Glu($OBu^t$) ; D-Thr($OBu^t$) ; D-Cys($OBu^t$) ; D-Ser($OR_1$) where $R_1$ is a sugar moiety ; an aza-amino acid ; D-His which may be substituted on the imidazole ring by a ($C_1$-$C_6$)alkyl or a benzyl group ; an aliphatic D-amino acid with a ($C_1$-$C_8$)alkyl or a ($C_3$-$C_6$)cycloalkyl side chain ; an aromatic D-amino acid ; D-cyclohexadienyl-Gly ; D-perhydronaphtyl-Ala; D-perhydrodiphenyl-Ala ; or a basic D-amino acid;
- A7 is a linear, branched or cyclic aliphatic L-amino acid of 3 to 20 carbon atoms which may be N-alpha-substituted by a ($C_1$-$C_4$)alkyl group optionally substituted by one or several fluorine atoms;
- A8 is a basic L-amino acid;

- Z is $GlyNH_2$ ; $azaGlyNH_2$ ; or a group $-NHR_2$ where $R_2$ is a $(C_1-C_4)$alkyl which may be substituted by an hydroxy or one or several fluorine atoms ; a $(C_3-C_6)$cycloalkyl ; or a heterocyclic radical selected from morpholinyl, pyrrolidinyl and piperidyl.

**4.** Use according to claim 3 wherein said peptide analogue has the formula (SEQ ID N° : 3):

pGlu-His-A3-Ser-A5-A6-A7-Arg-Pro-Z (II)

in which A7 is Leu, Tle, Nle, Hol, Npg, Cha or Ada, which may be N-alpha-substituted by a methyl or ethyl group optionally substituted by one or several fluorine atoms.

**5.** Use according to claim 3 wherein said peptide analogue has the formula (SEQ ID N° : 4) :

pGlu-His-A3-Ser-A5-A6-A7-Arg-Pro-Z (III)

in which:

- A3 and A5 are each independently Phe, Tyr, Trp, 2MeTrp, HPhe, HTyr, Nal, 1Nal, Bal, Pal, 4Pal, or pClPhe;
- A6 is (S)-spirolactam-Pro ; Gly; D-Pro ; D-Ser($OBu^t$) ; D-Asp($OBu^t$) ; D-Glu($OBu^t$) ; D-Thr($OBu^t$) ; D-Cys ($OBu^t$); D-His or D-His(Bzl); D-Ala, D-Leu, D-Tle, D-Nle, D-Hol, D-Npg or D-Cha ; D-Phe, D-HPhe, D-Tyr, D-HTyr, D-Trp, D-2MeTrp, D-Nal, D-1Nal, D-Bal, D-Pal, D-4Pal, or D-pClPhe; D-cyclohexadienyl-Gly ; D-perhydronaphtyl-Ala; D-perhydrodiphenyl-Ala ; or D-APhe optionally substituted by an aminotriazolyl group ;
- A7 is Leu, Npg or Cha, which may be N-alpha-substituted by a methyl group;
- Z is $GlyNH_2$ ; $azaGlyNH_2$ or $-NHC_2H_5$.

**6.** Use according to claim 3 wherein said peptide analogue has the formula (SEQ ID N° : 5):

pGlu-His-Trp-Ser-Tyr-A6-A7-Arg-Pro-Z (IV)

in which:

- A6 is (S)-spirolactam-Pro, D-Leu, D-Ala, D-Nal, D-Phe, D-Ser($OBu^t$) or D-Trp;
- A7 is Leu, MeLeu, Npg or MeNpg;
- Z is $GlyNH_2$ ; $azaGlyNH_2$ or $-NHC_2H_5$.

**7.** Use according to one of claims 3 to 6 wherein the peptide analogue is selected from the group consisting of leuprorelin, [$Npg^7$]-leuprorelin, triptorelin, [$Npg^7$]-triptorelin, goserelin, [$Npg^7$]-goserelin, buserelin and [$Npg^7$]-buserelin.

**8.** Use according to claim 7 wherein the peptide analogue is leuprorelin or [$Npg^7$]-leuprorelin.

**9.** Use according to claim 2 wherein said peptide analogue has the formula (SEQ ID N° : 6):

A1-A2-A3-A4-A5-A6-A7-A8-Pro-Z (I')

in which:

- A1 is pGlu ; D-pGlu ; Sar ; AcSar; Pro or a derivative thereof; Ser; D-Ser; Ac-D-Ser; Thr; D-Thr ; Ac-D-Thr ; or an aromatic D-amino acid which may be acylated;
- A2 is a direct bond or an aromatic D-amino acid;
- A3 is an aromatic L- or D-amino acid;
- A4 is Ala, Ser, D-Ser, MeSer, Ser($OBu^t$), Ser(OBzl) or Thr;
- A5 is an aromatic L-amino acid or a basic L- or D-amino acid;
- A6 is Gly ; (S)-spirolactam-Pro ; D-Pro ; D-Ser; D-Thr; D-Cys ; D-Met; D-Asn ; D-Pen ; D-(S-Me)Pen ; D-(S-Et)Pen ; D-Ser(OBu$^t$) ; D-Asp(OBu$^t$) ; D-Glu(O-Bu$^t$) ; D-Thr(O-Bu$^t$) ; D-Cys(O-Bu$^t$) ; D-Ser(O-$R_1$) where $R_1$ is a sugar moiety ; an aliphatic D-amino acid with a ($C_1$-$C_8$)alkyl or a ($C_3$-$C_6$)cycloalkyl side chain ; an aromatic D-amino acid ; D-cyclohexadienyl-Gly; D-perhydronaphthyl-Ala ; D-perhydrodiphenyl-Ala ; or a basic L- or D-amino acid;

- A7 is a linear, branched or cyclic aliphatic L-amino acid of 3 to 20 carbon atoms which may be N-alpha-substituted by a ($C_1$-$C_4$)alkyl group optionally substituted by one or several fluorine atoms;
- A8 is a basic L- or D-amino acid;
- Z is $GlyNH_2$ or D-$AlaNH_2$.

**10.** Use according to claim 9 wherein the peptide analogue has the formula (SEQ ID N° : 7):

Ac-D-Nal-D-pClPhe-D-Pal-Ser-A5-A6-A7-A8-Pro-D-$AlaNH_2$ (II')

in which:

- A5 is Tyr, HTyr, MeTyr, MeHTyr, NicLys or IprLys;
- A6 is (S)spirolactam-Pro, D-Arg, D-NicLys, D-IprLys, D-Cit, D-HCit or D-Asn;
- A7 is Leu, MeLeu, Npg or MeNpg;
- A8 is Arg, NicLys or IprLys.

**11.** Use according to claim 9 or 10 wherein the peptide analogue is selected from the group consisting of antide, [$Npg^7$]-antide, cetrorelix, [$Npg^7$]-cetrorelix, abarelix and [$Npg^7$]-abarelix.

**12.** Use according to one of claims 1 to 11 wherein the $\alpha$-cyclodextrin derivative is selected from the group consisting of methylated $\alpha$-cyclodextrin, hexakis(2,3,6-tri-O-methyl)-$\alpha$-cyclodextrin, carboxymethylated $\alpha$-cyclodextrin and phosphated $\alpha$-cyclodextrin.

**13.** Use according to claim 12 wherein the $\alpha$-cyclodextrin derivative is hexakis(2,3,6-tri-O-methyl)-$\alpha$-cyclodextrin.

**14.** A pharmaceutical composition for the gastrointestinal delivery by oral administration of a LH-RH peptide analogue which comprises a therapeutically effective amount of said peptide analogue in combination with $\alpha$-cyclodextrin or a derivative thereof.

**15.** The pharmaceutical composition according to claim 14 which further comprises excipients suitable for the gastrointestinal delivery of the peptide analogue.

**16.** The pharmaceutical composition according to claim 14 or 15 wherein said peptide analogue has the formula (SEQ ID N°: 1):

A1-A2-A3-A4-A5-A6-A7-A8-Pro-Z (A)

in which:

- A1 is pGlu ; D-pGlu ; Sar; AcSar; Pro or a derivative thereof ; Ser; D-Ser; Ac-D-Ser; Thr; D-Thr; Ac-D-Thr ; or an aromatic D-amino acid which may be acylated ;
- A2 is a direct bond ; His ; or an aromatic D-amino acid;
- A3 is an aromatic L- or D-amino acid;
- A4 is Ala, Ser, D-Ser, MeSer, Ser(OBu$^t$), Ser(OBzl) or Thr;
- A5 is an aromatic L-amino acid ; or a basic L- or D-amino acid;
- A6 is Gly; (S)-spirolactam-Pro ; D-Pro ; D-Ser; D-Thr; D-Cys ; D-Asn ; D-Met; D-Pen ; D-(S-Me)Pen ; D-(S-Et) Pen ; D-Ser(OBu$^t$) ; D-Asp(OBu$^t$) ; D-Glu(OBu$^t$) ; D-Thr(OBu$^t$) ; D-Cys(OBu$^t$) ; D-Ser(O$R_1$) where $R_1$ is a sugar moiety ; an aza-amino acid ; D-His which may be substituted on the imidazole ring by a ($C_1$-$C_6$)alkyl, a ($C_2$-$C_7$) acyl or a benzyl group ; an aliphatic D-amino acid with a ($C_1$-$C_8$)alkyl or a ($C_3$-$C_6$)cycloalkyl side chain ; an aromatic D-amino acid ; D-cyclohexadienyl-Gly; D-perhydronaphthyl-Ala ; D-perhydrodiphenyl-Ala ; or a basic L- or D-amino acid;

- A7 is a linear, branched or cyclic aliphatic L-amino acid of 3 to 20 carbon atoms which may be N-alpha-substituted by a $(C_1-C_4)$alkyl group optionally substituted by one or several fluorine atoms;
- A8 is a basic L- or D-amino acid ;
- Z is $GlyNH_2$ ; $D-AlaNH_2$ ; $azaGlyNH_2$ ; or a group $-NHR_2$ where $R_2$ is a $(C_1-C_4)$alkyl which may be substituted by an hydroxy or one or several fluorine atoms ; a $(C_3-C_6)$cycloalkyl ; or a heterocyclic radical selected from morpholinyl, pyrrolidinyl and piperidyl.

**17.** The pharmaceutical composition according to claim 16 wherein said peptide analogue has the formula (SEQ ID N° : 2):

A1-His-A3-A4-A5-A6-A7-A8-Pro-Z (I)

in which:

- A1 is pGlu, Sar or AcSar;
- A3 is an aromatic L-amino acid ;
- A4 is Ala, Ser, D-Ser, MeSer, $Ser(OBu^t)$, Ser(OBzl) or Thr;
- A5 is an aromatic L-amino acid ;
- A6 is Gly ; D-Pro ; (S)-spirolactam-Pro ; D-Ser; D-Thr; D-Cys ; D-Met; D-Pen ; D-(S-Me)Pen ; D-(S-Et)Pen ; $D-Ser(OBu^t)$ ; $D-Asp(OBu^t)$ ; $D-Glu(OBu^t)$ ; $D-Thr(OBu^t)$ ; $D-Cys(OBu^t)$ ; $D-Ser(OR_1)$ where $R_1$ is a sugar moiety ; an aza-amino acid ; D-His which may be substituted on the imidazole ring by a $(C_1-C_6)$alkyl or a benzyl group ; an aliphatic D-amino acid with a $(C_1-C_8)$alkyl or a $(C_3-C_6)$cycloalkyl side chain ; an aromatic D-amino acid ; D-cyclohexadienyl-Gly ; D-perhydronaphtyl-Ala ; D-perhydrodiphenyl-Ala ; or a basic D-amino acid ;
- A7 is a linear, branched or cyclic aliphatic L-amino acid of 3 to 20 carbon atoms which may be N-alpha-substituted by a $(C_1-C_4)$alkyl group optionally substituted by one or several fluorine atoms;
- A8 is a basic L-amino acid ;
- Z is $GlyNH_2$ ; $azaGlyNH_2$ ; or a group $-NHR_2$ where $R_2$ is a $(C_1-C_4)$alkyl which may be substituted by an hydroxy or one or several fluorine atoms ; a $(C_3-C_6)$cycloalkyl ; or a heterocyclic radical selected from morpholinyl, pyrrolidinyl and piperidyl.

**18.** The pharmaceutical composition according to claim 17 wherein said peptide analogue has the formula (SEQ ID N° : 3):

pGlu-His-A3-Ser-A5-A6-A7-Arg-Pro-Z (II)

in which A7 is Leu, Tle, Nle, Hol, Npg, Cha or Ada, which may be N-alpha-substituted by a methyl or ethyl group optionally substituted by one or several fluorine atoms.

**19.** The pharmaceutical composition according to claim 17 wherein said peptide analogue has the formula (SEQ ID N°: 4):

pGlu-His-A3-Ser-A5-A6-A7-Arg-Pro-Z (III)

in which:

- A3 and A5 are each independently Phe, Tyr, Trp, 2MeTrp, HPhe, HTyr, Nal, 1Nal, Bal, Pal, 4Pal, or pClPhe;
- A6 is (S)-spirolactam-Pro ; Gly; D-Pro ; $D-Ser(OBu^t)$ ; $D-Asp(OBu^t)$ ; $D-Glu(OBu^t)$ ; $D-Thr(OBu^t)$ ; $D-Cys(OBu^t)$; D-His or D-His(Bzl) ; D-Ala, D-Leu, D-Tle, D-Nle, D-Hol, D-Npg or D-Cha ; D-Phe, D-HPhe, D-Tyr, D-HTyr, D-Trp, D-2MeTrp, D-Nal, D-1Nal, D-Bal, D-Pal, D-4Pal, or D-pClPhe; D-cyclohexadienyl-Gly; D-perhydronaphtyl-Ala ; D-perhydrodiphenyl-Ala or D-APhe optionally substituted by an aminotriazolyl group;
- A7 is Leu, Npg or Cha, which may be N-alpha-substituted by a methyl group;
- Z is $GlyNH_2$, $azaGlyNH_2$ or $-NHC_2H_5$.

**20.** The pharmaceutical composition according to claim 17 wherein said peptide analogue has the formula (SEQ ID N° : 5):

pGlu-His-Trp-Ser-Tyr-A6-A7-Arg-Pro-Z (IV)

in which:

- A6 is (S)-spirolactam-Pro, D-Leu, D-Ala, D-Nal, D-Phe, D-Ser($OBu^t$) or D-Trp;
- A7 is Leu, MeLeu, Npg or MeNpg;
- Z is $GlyNH_2$, $azaGlyNH_2$ or $-NHC_2H_5$.

**21.** The pharmaceutical composition according to one of claims 17 to 20 wherein the peptide analogue is selected from the group consisting of leuprorelin, [$Npg^7$]-leuprorelin, triptorelin, [$Npg^7$]-triptorelin, goserelin, [$Npg^7$]-goserelin, buserelin and [$Npg^7$]-buserelin.

**22.** The pharmaceutical composition according to claim 21 wherein the peptide analogue is leuprorelin or [$Npg^7$]-leuprorelin.

**23.** The pharmaceutical composition according to claim 16 wherein said peptide analogue has the formula (SEQ ID N° : 6):

A1-A2-A3-A4-A5-A6-A7-A8-Pro-Z (I')

in which:

- A1 is pGlu ; D-pGlu ; Sar ; AcSar; Pro or a derivative thereof; Ser; D-Ser ; Ac-D-Ser ; Thr; D-Thr; Ac-D-Thr; or an aromatic D-amino acid which may be acylated;
- A2 is a direct bond or an aromatic D-amino acid;
- A3 is an aromatic L- or D-amino acid;
- A4 is Ala, Ser, D-Ser, MeSer, Ser($OBu^t$), Ser(OBzl) or Thr;
- A5 is an aromatic L-amino acid or a basic L- or D-amino acid;
- A6 is Gly ; (S)-spirolactam- Pro ; D-Pro ; D-Ser; D-Thr ; D-Cys ; D-Met ; D-Asn ; D-Pen ; D-(S-Me)Pen ; D-(S-Et)Pen ; D-Ser($OBu^t$) ; D-Asp($OBu^t$) ; D-Glu(O-$Bu^t$) ; D-Thr(O-$Bu^t$) ; D-Cys(O-$Bu^t$) ; D-Ser(O-$R_1$) where $R_1$ is a sugar moiety; an aliphatic D-amino acid with a ($C_1$-$C_8$)alkyl or a ($C_3$-$C_6$)cycloalkyl side chain ; an aromatic D-amino acid ; D-cyclohexadienyl-Gly ; D-perhydronaphthyl-Ala ; D-perhydrodiphenyl-Ala ; or a basic L- or D-amino acid;
- A7 is a linear, branched or cyclic aliphatic L-amino acid of 3 to 20 carbon atoms which may be N-alpha-substituted by a ($C_1$-$C_4$)alkyl group optionally substituted by one or several fluorine atoms;
- A8 is a basic L- or D-amino acid;
- Z is $GlyNH_2$ or $D\text{-}AlaNH_2$.

**24.** The pharmaceutical composition according to claim 23 wherein the peptide analogue has the formula (SEQ ID N° : 7):

Ac-D-Nal-D-pClPhe-D-Pal-Ser-A5-A6-A7-A8-Pro-$D\text{-}AlaNH_2$ (II')

in which:

- A5 is Tyr, HTyr, MeTyr, MeHTyr, NicLys or IprLys;
- A6 is (S)spirolactam-Pro, D-Arg, D-NicLys, D-IprLys, D-Cit, D-HCit or D-Asn;
- A7 is Leu, MeLeu, Npg or MeNpg;
- A8 is Arg, NicLys or IprLys.

**25.** The pharmaceutical composition according to claim 23 or 24 wherein the peptide analogue is selected from the group consisting of antide, [$Npg^7$]-antide, cetrorelix, [$Npg^7$]-cetrorelix, abarelix and [$Npg^7$]-abarelix.

**26.** The pharmaceutical composition according to one of claims 14 to 25 wherein the $\alpha$-cyclodextrin derivative is se-

lected from the group consisting of methylated α-cyclodextrin, hexakis(2,3,6-tri-O-methyl)-α-cyclodextrin, carboxymethylated α-cyclodextrin and phosphated α-cyclodextrin.

**27.** The pharmaceutical composition according to claim 26 wherein the α-cyclodextrin derivative is hexakis(2,3,6-tri-O-methyl)-α-cydodextrin.

**28.** The pharmaceutical composition according to one of claims 14 to 27 which further comprises a protease inhibitor and/or an absorption enhancer.

**Patentansprüche**

**1.** Verwendung von α-Cyclodextrin oder einem Derivat davon zur Herstellung einer pharmazeutischen Zusammensetzung zur oralen Verabreichung eines LH-RH-Peptidanalogon oder eines seiner pharmazeutisch verträglichen Salze.

**2.** Verwendung gemäß Anspruch 1, wobei das Peptidanalogon die Formel (SEQ ID No.1) hat:

A1-A2-A3-A4-A5-A6-A7-A8-Pro-Z (A)

wobei:

A1 pGlu; D-pGlu; Sar; AcSar; Pro oder ein Derivat davon; Ser; D-Ser; Ac-D-Ser; Thr; D-Thr; Ac-D-Thr; oder eine aromatische D-Aminosäure, die acyliert sein kann, ist;
A2 eine direkte Bindung; His; oder eine aromatische D-Aminosäure ist;
A3 eine aromatische L- oder D-Aminosäure ist;
A4 Ala, Ser, D-Ser, MeSer, Ser($OBu^t$), Ser(OBzl) oder Thr ist;
A5 eine aromatische L-Aminosäure oder eine basische L-oder D-Aminosäure ist;
A6 Gly; (S)-Spirolactam-Pro; D-Pro; D-Ser; D-Thr; D-Cys; D-Met; D-Asn; D-Pen; D-(S-Me)Pen; D-(S-Et)Pen; D-Ser($OBu^t$); D-Asp($OBu^t$) ; D-Glu($OBu^t$), D-Thr($OBu^t$) ; D-Cys($OBu^t$); D-Ser($OR_1$), wobei $R_1$ eine Zuckergruppe ist; eine Aza-Aminosäure; D-His, das am Imidazolring mit einer ($C_1$-$C_6$)Alkyl-, einer ($C_2$-$C_7$)Acyl- oder einer Benzylgruppe substituiert sein kann; eine aliphatische D-Aminosäure mit einer ($C_1$-$C_8$)Alkyl- oder einer ($C_3$-$C_6$)Cycloalkyl-Seitenkette; eine aromatische D-Aminosäure; D-Cyclohexadienyl-Gly; D-Perhydronaphthyl-Ala; D-Perhydrodiphenyl-Ala; oder eine basische L- oder D-Aminosäure ist;
A7 eine gerade, verzweigte oder zyklische aliphatische L-Aminosäure mit 3 bis 20 Kohlenstoffatomen ist, die mit einer ($C_1$-$C_4$)Alkylgruppe, die ggf. mit einem oder mehreren Fluoratomen substituiert ist, N-alpha-substituiert sein kann;
A8 eine basische L- oder D-Aminosäure ist;
Z $GlyNH_2$; D-$AlaNH_2$; aza$GlyNH_2$; oder eine -$NHR_2$-Gruppe,
wobei $R_2$ ($C_1$-$C_4$)Alkyl ist, das mit Hydroxy- oder einem oder mehreren Fluoratomen substituiert sein kann; ($C_3$-$C_6$)Cycloalkyl; oder ein heterozyklischer Rest, ausgewählt aus Morpholinyl, Pyrrolidinyl und Piperidyl, ist.

**3.** Verwendung gemäß Anspruch 2, wobei das Peptidanalogon die Formel (SEQ ID No.2) hat:

A1-His-A3-A4-A5-A6-A7-A8-Pro-Z (I)

wobei:

A1 pGlu, Sar oder AcSar ist;
A3 eine aromatische L-Aminosäure ist;
A4 Ala, Ser, D-Ser, MeSer, Ser($OBu^t$), Ser(OBzl) oder Thr ist;
A5 eine aromatische L-Aminosäure ist;
A6 Gly; D-Pro; (S)-Spirolactam-Pro; D-Ser; D-Thr; D-Cys; D-Met; D-Pen; D-(S-Me)Pen; D-(S-Et)Pen; D-Ser ($OBu^t$); D-Asp($OBu^t$); D-Glu($OBu^t$); D-Thr($OBu^t$); D-Cys($OBu^t$); D-Ser($OR_1$), wobei $R_1$ eine Zuckergruppe ist; eine Aza-Aminosäure; D-His, das am Imidazolring mit einer ($C_1$-$C_6$)Alkyl- oder einer Benzylgruppe substituiert sein kann; eine aliphatische D-Aminosäure mit einer ($C_1$-$C_8$)Alkyl- oder einer ($C_3$-$C_6$)Cycloalkyl-Seitenkette;

eine aromatische D-Aminosäure; D-Cyclohexadienyl-Gly; D-Perhydronaphthyl-Ala; D-Perhydrodiphenyl-Ala; oder eine basische D-Aminosäure ist;
A7 eine gerade, verzweigte oder zyklische aliphatische L-Aminosäure mit 3 bis 20 Kohlenstoffatomen ist, die mit einer ($C_1$-$C_4$)Alkylgruppe, die ggf. mit einem oder mehreren Fluoratomen substituiert ist, N-alpha-substituiert sein kann;
A8 eine basische L-Aminosäure ist;
Z $GlyNH_2$; $azaGlyNH_2$; oder eine $-NHR_2$-Gruppe, wobei $R_2$ ($C_1$-$C_4$)Alkyl ist, das mit Hydroxy- oder einem oder mehreren Fluoratomen substituiert sein kann; ($C_3$-$C_6$)Cycloalkyl; oder ein heterozyklischer Rest, ausgewählt aus Morpholinyl, Pyrrolidinyl und Piperidyl, ist.

**4.** Verwendung gemäß Anspruch 3, wobei das Peptidanalogon die Formel (SEQ ID No.3) hat:

pGlu-His-A3-Ser-A5-A6-A7-Arg-Pro-Z (II)

wobei A7 Leu, Tle, Nle, Hol, Npg, Cha oder Ada ist, die mit einer Methyl- oder Ethylgruppe, die ggf. mit einem oder mehreren Fluoratomen substituiert sind, N-alpha-substituiert sein können.

**5.** Verwendung gemäß Anspruch 3, wobei das Peptidanalogon die Formel (SEQ ID No.4) hat:

pGlu-His-A3-Ser-A5-A6-A7-Arg-Pro-Z (III)

wobei:

A3 und A5 unabhängig Phe, Tyr, Trp, 2MeTrp, HPhe, HTyr, Nal, 1Nal, Bal, Pal, 4Pal oder pClPhe sind;
A6 (S)-Spirolactam-Pro; Gly; D-Pro; D-Ser($OBu^t$) ; D-Asp($OBu^t$); D-Glu($OBu^t$); D-Thr($OBu^t$) ; D-Cys($OBu^t$) ; D-His oder D-His(Bzl); D-Ala, D-Leu, D-Tle, D-Nle, D-Hol, D-Npg oder D-Cha; D-Phe, D-HPhe, D-Tyr, D-HTyr, D-Trp, D-2MeTrp, D-Nal, D-1Nal, D-Bal, D-Pal, D-4Pal oder D-pClPhe; D-Cyclohexadienyl-Gly; D-Perhydronaphthyl-Ala; D-Perhydrodiphenyl-Ala; oder D-APhe ist, ggf. mit einer Aminotriazolylgruppe substituiert;
A7 Leu, Npg oder Cha, die mit einer Methylgruppe N-alpha-substituiert sein können, ist;
Z $GlyNH_2$; $azaGlyNH_2$ oder $-NHC_2H_5$ ist.

**6.** Verwendung gemäß Anspruch 3, wobei das Peptidanalogon die Formel (SEQ ID No.5) hat:

pGlu-His-Trp-Ser-Tyr-A6-A7-Arg-Pro-Z (IV)

wobei:

A6 (S)-Spirolactam-Pro, D-Leu, D-Ala, D-Nal, D-Phe, D-Ser(OBut) oder D-Trp ist;
A7 Leu, MeLeu, Npg oder MeNpg ist;
Z $GlyNH_2$; $azaGlyNH_2$ oder $-NHC_2H_5$ ist.

**7.** Verwendung gemäß einem der Ansprüche 3 bis 6, wobei das Peptidanalogon aus der Gruppe ausgewählt ist, die aus Leuprorelin, [$Npg^7$]-Leuprorelin, Triptorelin, [$Npg^7$]-Triptorelin, Goserelin, [$Npg^7$]-Goserelin, Buserelin und [$Npg^7$]-Buserelin besteht.

**8.** Verwendung gemäß Anspruch 7, wobei das Peptidanalogon Leuprorelin oder [$Npg^7$]-Leuprorelin ist.

**9.** Verwendung gemäß Anspruch 2, wobei das Peptidanalogon die Formel (SEQ ID No.6) hat:

A1-A2-A3-A4-A5-A6-A7-A8-Pro-Z (I')

wobei:

A1 pGlu; D-pGlu; Sar; AcSar; Pro oder ein Derivat davon; Ser; D-Ser; Ac-D-Ser; Thr; D-Thr; Ac-D-Thr; oder

eine aromatische D-Aminosäure, die acyliert sein kann, ist;
A2 eine direkte Bindung oder eine aromatische D-Aminosäure ist;
A3 eine aromatische L- oder D-Aminosäure ist;
A4 Ala, Ser, D-Ser, MeSer, Ser($OBu^t$), Ser(OBzl) oder Thr ist;
A5 eine aromatische L-Aminosäure oder eine basische L-oder D-Aminosäure ist;
A6 Gly; (S)-Spirolactam-Pro; D-Pro; D-Ser; D-Thr; D-Cys; D-Met; D-Asn; D-Pen; D-(S-Me)Pen; D-(S-Et)Pen; D-Ser($OBu^t$) ; D-Asp($OBu^t$) ; D-Glu($OBu^t$) ; D-Thr($OBu^t$) ; D-Cys ($OBu^t$) ; D-Ser (O-$R_1$), wobei $R_1$ eine Zukkergruppe ist; eine aliphatische D-Aminosäure mit einer ($C_1$-$C_8$)Alkyl- oder einer ($C_3$-$C_6$)Cycloalkyl-Seitenkette; eine aromatische D-Aminosäure; D-Cyclohexadienyl-Gly; D-Perhydronaphthyl-Ala; D-Perhydrodiphenyl-Ala; oder eine basische L- oder D-Aminosäure ist;
A7 eine gerade, verzweigte oder zyklische aliphatische L-Aminosäure mit 3 bis 20 Kohlenstoffatomen ist, die mit einer ($C_1$-$C_4$)Alkylgruppe, die ggf. mit einem oder mehreren Fluoratomen substituiert ist, N-alpha-substituiert sein kann;
A8 eine basische L- oder D-Aminosäure ist;
Z $GlyNH_2$ oder D-$AlaNH_2$ ist.

**10.** Verwendung gemäß Anspruch 9, wobei das Peptidanalogon die Formel (SEQ ID No.7) hat:

Ac-D-Nal-D-pClPhe-D-Pal-Ser-A5-A6-A7-A8-Pro-D-$AlaNH_2$ (II')

wobei:

A5 Tyr, HTyr, MeTyr, MeHTyr, NicLys oder IprLys ist;
A6 (S)-Spirolactam-Pro, D-Arg, D-NicLys, D-IprLys, D-Cit, D-HCit oder D-Asn ist;
A7 Leu, MeLeu, Npg oder MeNpg ist;
A8 Arg, NicLys oder IprLys ist.

**11.** Verwendung gemäß Anspruch 9 oder 10, wobei das Peptidanalogon aus der Gruppe ausgewählt ist, die aus Antid, [$Npg^7$]-Antid, Cetrorelix, [$Npg^7$]-Cetrorelix, Abarelix und [$Npg^7$]-Abarelix besteht.

**12.** Verwendung gemäß einem der Ansprüche 1 bis 11, wobei das α-Cyclodextrinderivat aus der Gruppe ausgewählt ist, die aus methyliertem α-Cyclodextrin, Hexakis(2,3,6-tri-Omethyl)-α-cyclodextrin, carboxymethyliertem α-Cyclodextrin und phosphatisiertem α-Cyclodextrin besteht.

**13.** Verwendung gemäß Anspruch 12, wobei das α-Cyclodextrinderivat Hexakis(2,3,6-tri-O-methyl)-α-cyclodextrin ist.

**14.** Pharmazeutische Zusammensetzung zur gastrointestinalen Aufnahme durch orale Verabreichung eines LH-RH-Peptidanalogon, die eine therapeutisch wirksame Menge des Peptidanalogon in Kombination mit α-Cyclodextrin oder einem Derivat davon umfaßt.

**15.** Pharmazeutische Zusammensetzung gemäß Anspruch 14, die außerdem geeignete Exzipienten zur gastrointestinalen Aufnahme des Peptidanalogon umfaßt.

**16.** Pharmazeutische Zusammensetzung gemäß Anspruch 14 oder 15, wobei das Peptidanalogon die Formel (SEQ ID No.1) hat:

A1-A2-A3-A4-A5-A6-A7-A8-Pro-Z (A)

wobei:

A1 pGlu; D-pGlu; Sar; AcSar; Pro oder ein Derivat davon; Ser; D-Ser; Ac-D-Ser; Thr; D-Thr; Ac-D-Thr; oder eine aromatische D-Aminosäure, die acyliert sein kann, ist;
A2 eine direkte Bindung; His; oder eine aromatische D-Aminosäure ist;
A3 eine aromatische L- oder D-Aminosäure ist;
A4 Ala, Ser, D-Ser, MeSer, Ser($OBu^t$), Ser(OBzl) oder Thr ist;

A5 eine aromatische L-Aminosäure; oder eine basische L-oder D-Aminosäure ist;
A6 Gly; (S)-Spirolactam-Pro; D-Pro; D-Ser; D-Thr; D-Cys; D-Met; D-Asn; D-Pen; D-(S-Me)Pen; D-(S-Et)Pen; D-Ser($OBu^t$) ; D-Asp($OBu^t$); D-Glu($OBu^t$); D-Thr($OBu^t$); D-Cys($OBu^t$); D-Ser($OR_1$), wobei $R_1$ eine Zuckergruppe ist; eine Aza-Aminosäure; D-His, das am Imidazolring mit einer ($C_1$-$C_6$)Alkyl-, einer ($C_2$-$C_7$)Acyl- oder einer Benzylgruppe substituiert sein kann; eine aliphatische D-Aminosäure mit einer ($C_1$-$C_8$)Alkyl- oder einer ($C_3$-$C_6$) Cycloalkyl-Seitenkette; eine aromatische D-Aminosäure; D-Cyclohexadienyl-Gly; D-Perhydronaphthyl-Ala; D-Perhydrodiphenyl-Ala; oder eine basische L- oder D-Aminosäure ist;
A7 eine gerade, verzweigte oder zyklische aliphatische L-Aminosäure mit 3 bis 20 Kohlenstoffatomen ist, die mit einer ($C_1$-$C_4$)Alkylgruppe, die ggf. mit einem oder mehreren Fluoratomen substituiert ist, N-alpha-substituiert sein kann; A8 eine basische L- oder D-Aminosäure ist;
Z $GlyNH_2$; D-$AlaNH_2$; aza$GlyNH_2$; oder eine -$NHR_2$-Gruppe ist, wobei $R_2$ ($C_1$-$C_4$)Alkyl ist, das mit Hydroxy oder einem oder mehreren Fluoratomen substituiert sein kann; ($C_3$-$C_6$)Cycloalkyl; oder ein heterozyklischer Rest, ausgewählt aus Morpholinyl, Pyrrolidinyl und Piperidyl.

**17.** Pharmazeutische Zusammensetzung gemäß Anspruch 16, wobei das Peptidanalogon die Formel (SEQ ID No.2) hat:

A1-His-A3-A4-A5-A6-A7-A8-Pro-Z (I)

wobei:

A1 pGlu, Sar oder AcSar ist;
A3 eine aromatische L-Aminosäure ist;
A4 Ala, Ser, D-Ser, MeSer, Ser($OBu^t$), Ser(OBzl) oder Thr ist;
A5 eine aromatische L-Aminosäure ist;
A6 Gly; D-Pro; (S)-Spirolactam-Pro; D-Ser; D-Thr; D-Cys; D-Met; D-Pen, D-(S-Me)Pen; D-(S-Et)Pen; D-Ser ($OBu^t$); D-Asp($OBu^t$); D-Glu($OBu^t$); D-Thr($OBu^t$); D-Cys($OBu^t$) ; D-Ser($OR_1$), wobei $R_1$ eine Zuckergruppe ist; eine Aza-Aminosäure; D-His, das am Imidazolring mit einer ($C_1$-$C_6$)Alkyl- oder einer Benzylgruppe substituiert sein kann; eine aliphatische D-Aminosäure mit einer ($C_1$-$C_8$)Alkyl- oder einer ($C_3$-$C_6$)Cycloalkyl-Seitenkette; eine aromatische D-Aminosäure; D-Cyclohexadienyl-Gly; D-Perhydronaphthyl-Ala; D-Perhydrodiphenyl-Ala; oder eine basische D-Aminosäure ist;
A7 eine gerade, verzweigte oder zyklische aliphatische L-Aminosäure mit 3 bis 20 Kohlenstoffatomen ist, die mit einer ($C_1$-$C_4$)Alkylgruppe, die ggf. mit einem oder mehreren Fluoratomen substituiert ist, N-alpha-substituiert sein kann;
A8 eine basische L-Aminosäure ist;
Z $GlyNH_2$; aza$GlyNH_2$; oder eine -$NHR_2$-Gruppe, wobei $R_2$ ein ($C_1$-$C_4$)Alkyl ist, das mit Hydroxy oder einem oder mehreren Fluoratomen substituiert sein kann; ($C_3$-$C_6$)Cycloalkyl; oder ein heterozyklischer Rest, ausgewählt aus Morpholinyl, Pyrrolidinyl und Piperidyl, ist.

**18.** Pharmazeutische Zusammensetzung gemäß Anspruch 17, wobei das Peptidanalogon die Formel (SEQ ID No.3) hat:

pGlu-His-A3-Ser-A5-A6-A7-Arg-Pro-Z (II)

wobei A7 Leu, Tle, Nle, Hol, Npg, Cha oder Ada ist, die mit einer Methyl- oder Ethylgruppe, die ggf. mit einem oder mehreren Fluoratomen substituiert sind, N-alpha-substituiert sein können.

**19.** Pharmazeutische Zusammensetzung gemäß Anspruch 17, wobei das Peptidanalogon die Formel (SEQ ID No.4) hat:

pGlu-His-A3-Ser-A5-A6-A7-Arg-Pro-Z (III)

wobei:

A3 und A5 unabhängig Phe, Tyr, Trp, 2MeTrp, HPhe, HTyr, Nal, 1Nal, Bal, Pal, 4Pal oder pClPhe sind;

A6 (S)-Spirolactam-Pro; Gly; D-Pro; D-Ser(OBu[t]); D-Asp(OBu[t]) ; D-Glu(OBu[t]); D-Thr(OBu[t]); D-Cys(OBu[t]); D-His oder D-His(Bzl); D-Ala, D-Leu, D-Tle, D-Nle, D-Hol, D-Npg oder D-Cha; D-Phe, D-HPhe, D-Tyr, D-HTyr, D-Trp, D-2MeTrp, D-Nal, D-lNal, D-Bal, D-Pal, D-4Pal oder D-pClPhe; D-Cyclohexadienyl-Gly; D-Perhydronaphthyl-Ala; D-Perhydrodiphenyl-Ala oder D-APhe ist, ggf. mit einer Aminotriazolylgruppe substituiert;
A7 Leu, Npg oder Cha, die mit einer Methylgruppe N-alpha-substituiert sein können, ist;
Z $GlyNH_2$; $azaGlyNH_2$ oder $-NHC_2H_5$ ist.

**20.** Pharmazeutische Zusammensetzung gemäß Anspruch 17, wobei das Peptidanalogon die Formel (SEQ ID No.5) hat:

pGlu-His-Trp-Ser-Tyr-A6-A7-Arg-Pro-Z (IV)

wobei:

A6 (S)-Spirolactam-Pro, D-Leu, D-Ala, D-Nal, D-Phe, D-Ser(OBu[t]) oder D-Trp ist;
A7 Leu, MeLeu, Npg oder MeNpg ist;
Z $GlyNH_2$; $azaGlyNH_2$ oder $-NHC_2H_5$ ist.

**21.** Pharmazeutische Zusammensetzung gemäß einem der Ansprüche 17 bis 20, wobei das Peptidanalogon aus der Gruppe ausgewählt ist, die aus Leuprorelin, [$Npg^7$]-Leuprorelin, Triptorelin, [$Npg^7$]-Triptorelin, Goserelin, [$Npg^7$]-Goserelin, Buserelin und [$Npg^7$]-Buserelin besteht.

**22.** Pharmazeutische Zusammensetzung gemäß Anspruch 21, wöbei das Peptidanalogon Leuprorelin oder [$Npg^7$]-Leuprorelin ist.

**23.** Pharmazeutische Zusammensetzung gemäß Anspruch 16, wobei das Peptidanalogon die Formel (SEQ ID No.6) hat:

A1-A2-A3-A4-A5-A6-A7-A8-Pro-Z (I')

wobei:

A1 pGlu; D-pGlu; Sar; AcSar; Pro oder ein Derivat davon; Ser; D-Ser; Ac-D-Ser; Thr; D-Thr; Ac-D-Thr; oder eine aromatische D-Aminosäure, die acyliert sein kann, ist;
A2 eine direkte Bindung oder eine aromatische D-Aminosäure ist;
A3 eine aromatische L- oder D-Aminosäure ist;
A4 Ala, Ser, D-Ser, MeSer, Ser(OBu[t]), Ser(OBzl) oder Thr ist;
A5 eine aromatische L-Aminosäure oder eine basische L-oder D-Aminosäure ist;
A6 Gly; (S)-Spirolactam-Pro; D-Pro; D-Ser; D-Thr; D-Cys; D-Met; D-Asn; D-Pen; D-(S-Me)Pen; D-(S-Et)Pen; D-Ser(OBu[t]) ; D-Asp(OBu[t]); D-Glu(OBu[t]) ; D-Thr(OBu[t]); D-Cys(OBu[t]) ; D-Ser(O-$R_1$), wobei $R_1$ eine Zuckergruppe ist; eine aliphatische D-Aminosäure mit einer ($C_1$-$C_8$)Alkyl- oder einer ($C_3$-$C_6$)Cycloalkyl-Seitenkette; eine aromatische D-Aminosäure; D-Cyclohexadienyl-Gly; D-Perhydronaphthyl-Ala; D-Perhydrodiphenyl-Ala; oder eine basische L- oder D-Aminosäure ist;
A7 eine gerade, verzweigte oder zyklische aliphatische L-Aminosäure mit 3 bis 20 Kohlenstoffatomen ist, die mit einer ($C_1$-$C_4$)Alkylgruppe, die ggf. mit einem oder mehreren Fluoratomen substituiert ist, N-alpha-substituiert sein kann;
A8 eine basische L- oder D-Aminosäure ist;
Z $GlyNH_2$ oder D-$AlaNH_2$ ist.

**24.** Pharmazeutische Zusammensetzung gemäß Anspruch 23, wobei das Peptidanalogon die Formel (SEQ ID No.7) hat:

Ac-D-Nal-D-pClPhe-D-Pal-Ser-A5-A6-A7-A8-Pro-D-$AlaNH_2$ (II')

wobei:

A5 Tyr, HTyr, MeTyr, MeHTyr, NicLys oder IprLys ist;
A6 (S)-Spirolactam-Pro, D-Arg, D-NicLys, D-IprLys, D-Cit, D-HCit oder D-Asn ist;
A7 Leu, MeLeu, Npg oder MeNpg ist;
A8 Arg, NicLys oder IprLys ist.

**25.** Pharmazeutische Zusammensetzung gemäß Anspruch 23 oder 24, wobei das Peptidanalogon aus der Gruppe ausgewählt ist, die aus Antid, [$Npg^7$]-Antid, Cetrorelix, [$Npg^7$]-Cetrorelix, Abarelix und [$Npg^7$]-Abarelix besteht.

**26.** Pharmazeutische Zusammensetzung gemäß einem der Ansprüche 14 bis 25, wobei das α-Cyclodextrinderivat ausgewählt ist aus der Gruppe, die aus methyliertem α-Cyclodextrin, Hexakis(2,3,6-tri-O-methyl)-α-cyclodextrin, carboxymethyliertem α-Cyclodextrin und phosphatisiertem α-Cyclodextrin besteht.

**27.** Pharmazeutische Zusammensetzung gemäß Anspruch 26, wobei das α-Cyclodextrinderivat Hexakis(2,3,6-tri-O-methyl)-α-cyclodextrin ist.

**28.** Pharmazeutische Zusammensetzung gemäß einem der Ansprüche 14 bis 27, die außerdem einen Proteaseinhibitor und/oder einen Absorptionsverstärker umfaßt.

## Revendications

**1.** Utilisation de l'α-cyclodextrine ou d'un dérivé de celle-ci pour la préparation d'une composition pharmaceutique pour l'administration orale d'un analogue peptidique de la LH-RH ou de l'un de ses sels pharmaceutiquement acceptables.

**2.** Utilisation selon la revendication 1, où ledit analogue peptidique a la formule (SEQ ID N° : 1):

A1-A2-A3-A4-A5-A6-A7-A8-Pro-Z (A)

où:

- A1 est pGlu ; D-pGlu ; Sar ; AcSar ; Pro ou l'un de ses dérivés ; Ser ; D-Ser ; Ac-D-Ser ; Thr ; D-Thr ; Ac-D-Thr ; ou un D-aminoacide aromatique qui peut être acylé ;
- A2 est une liaison directe ; His ; ou un D-aminoacide aromatique ;
- A3 est un L- ou D-aminoacide aromatique ;
- A4 est Ala, Ser, D-Ser, MeSer, Ser($OBu^t$), Ser(OBzl) ou Thr ;
- A5 est un L-aminoacide aromatique ou un L- ou D- aminoacide basique ;
- A6 est Gly ; (S)-spirolactame-Pro ; D-Pro ; D-Ser ; D-Thr ; D-Cys ; D-Met ; D-Asn ; D-Pen ; D-(S-Me)Pen ; D-(S-Et)Pen ; D-Ser($OBu^t$) ; D-Asp($OBu^t$) ; D-Glu($OBu^t$) ; D-Thr($OBu^t$) ; D-Cys($OBu^t$) ; D-Ser($OR_1$) où $R_1$ est une entité glucidique ; un aza-aminoacide ; D-His qui peut être substitué sur le cycle imidazole par un groupe alkyle en $C_1$-$C_6$, acyle en $C_2$-$C_7$ ou benzyle ; un D-aminoacide aliphatique avec une chaîne latérale alkyle en $C_1$-$C_8$ ou cycloalkyle en $C_3$-$C_6$ ; un D-aminoacide aromatique; D-cyclohexadiényl-Gly ; D-perhydronaphtyl-Ala; D-perhydrodiphényl-Ala; ou un L- ou D-aminoacide basique ;
- A7 est un L-aminoacide aliphatique, linéaire, ramifié ou cyclique de 3 à 20 atomes de carbone qui peut être N-alpha-substitué par un groupe alkyle en $C_1$-$C_4$ éventuellement substitué par un ou plusieurs atomes de fluor ;
- A8 est un L- ou D-aminoacide basique ;
- Z est $GlyNH_2$ ; D-$AlaNH_2$ ; aza$GlyNH_2$ ; ou un groupe -$NHR_2$ où $R_2$ est un alkyle en $C_1$-$C_4$ qui peut être substitué par un hydroxyle ou un ou plusieurs atomes de fluor ; un cycloalkyle en $C_3$-$C_6$ ; ou un radical hétérocyclique choisi parmi morpholinyle, pyrrolidinyle et pipéridyle.

**3.** Utilisation selon la revendication 2 où ledit analogue peptidique a la formule (SEQ ID N° : 2):

A1-His-A3-A4-A5-A6-A7-A8-Pro-Z (I)

où :

- A1 est pGlu, Sar ou AcSar;
- A3 est un L-aminoacide aromatique ;
- A4 est Ala, Ser, D-Ser, MeSer, Ser($OBu^t$), Ser(OBzl) ou Thr ;
- A5 est un L-aminoacide aromatique ;
- A6 est Gly ; D-Pro ; (S)-spirolactame-Pro ; D-Ser ; D-Thr ; D-Cys ; D-Met ; D-Pen ; D-(S-Me)Pen ; D-(S-Et) Pen ; D-Ser($OBu^t$) ; D-Asp($OBu^t$) ; D-Glu($OBu^t$) ; D-Thr($OBu^t$) ; D-Cys($OBu^t$) ; D-Ser($OR_1$) où $R_1$ est une entité glucidique; un aza-aminoacide ; D-His qui peut être substitué sur le cycle imidazole par un alkyle en $C_1$-$C_6$, ou un groupe benzyle; un D-aminoacide aliphatique avec une chaîne latérale alkyle en $C_1$-$C_8$ ou cycloalkyle en $C_3$-$C_6$ ; un D-aminoacide aromatique ; D-cyclohexadiényl-Gly ; D-perhydronaphtyl-Ala ; D-perhydrodiphényl-Ala ; ou un D-aminoacide basique ;
- A7 est un L-aminoacide aliphatique, linéaire, ramifié ou cyclique de 3 à 20 atomes de carbone qui peut être N-alpha-substitué par un groupe alkyle en $C_1$-$C_4$ éventuellement substitué par un ou plusieurs atomes de fluor ;
- A8 est un L-aminoacide basique ;
- Z est $GlyNH_2$ ; $azaGlyNH_2$ ; ou un groupe $-NHR_2$ où $R_2$ est un alkyle en $C_1$-$C_4$ qui peut être substitué par un hydroxyle ou un ou plusieurs atomes de fluor ; un cycloalkyle en $C_3$-$C_6$ ; ou un radical hétérocyclique choisi parmi morpholinyle, pyrrolidinyle et pipéridyle.

**4.** Utilisation selon la revendication 3, où ledit analogue peptidique a la formule (SEQ ID N° : 3):

pGlu-His-A3-Ser-A5-A6-A7-Arg-Pro-Z (II)

où A7 est Leu, Tle, Nle, Hol, Npg, Cha ou Ada, qui peut être N-alpha-substitué par un groupe méthyle ou éthyle éventuellement substitué par un ou plusieurs atomes de fluor.

**5.** Utilisation selon la revendication 3, où ledit analogue peptidique a la formule (SEQ ID N° : 4):

pGlu-His-A3-Ser-A5-A6-A7-Arg-Pro-Z (III)

où

- A3 et A5 sont chacun indépendamment Phe, Tyr, Trp, 2MeTrp, HPhe, HTyr, Nal, 1Nal, Bal, Pal, 4Pal ou pClPhe;
- A6 est (S)-spirolactame-Pro; Gly ; D-Pro; D-Ser($OBu^t$) ; D-Asp($OBu^t$) ; D-Glu($OBu^t$) ; D-Thr($OBu^t$) ; D-Cys ($OBu^t$) ; D-His ou D-His(Bzl); D-Ala, D-Leu, D-Tle, D-Nle, D-Hol, D-Npg ou D-Cha; D-Phe, D-HPhe, D-Tyr, D-HTyr, D-Trp, D-2MeTrp, D-Nal, D-lNal, D-Bal, D-Pal, D-4Pal ou D-pClPhe; D-cyclohexadiényl-Gly, D-perhydronaphtyl-Ala; D-perhydrodiphényl-Ala ; ou D-APhe éventuellement substitué par un groupe aminotriazolyle;
- A7 est Leu, Npg ou Cha, qui peut être N-alpha-substitué par un groupe méthyle ;
- Z est $GlyNH_2$ ; $azaGlyNH_2$ ou $-NHC_2H_5$.

**6.** Utilisation selon la revendication 3, où ledit analogue peptidique a la formule (SEQ ID N° : 5):

pGlu-His-Trp-Ser-Tyr-A6-A7-Arg-Pro-Z (IV)

où:

- A6 est (S)-spirolactame-Pro, D-Leu, D-Ala, D-Nal, D-Phe, D-Ser($OBu^t$) ou D-Trp ;
- A7 est Leu, MeLeu, Npg ou MeNpg ;
- Z est $GlyNH_2$ ; $azaGlyNH_2$ ou $-NHC_2H_5$.

**7.** Utilisation selon l'une des revendications 3 à 6, où l'analogue peptidique est choisi dans le groupe consistant en la leuproréline, la [$Npg^7$]-leuproréline, la triptoréline, la [$Npg^7$]-triptoréline, la goséréline, la [$Npg^7$]-goséréline, la buséréline et la [$Npg^7$]-buséréline.

**8.** Utilisation selon la revendication 7, où l'analogue peptidique est la leuproréline ou la [$Npg^7$]-leuproréline.

**9.** Utilisation selon la revendication 2, où ledit analogue peptidique a la formule (SEQ ID N° : 6):

A1-A2-A3-A4-A5-A6-A7-A8-Pro-Z (I')

où:

- A1 est pGlu ; D-pGlu ; Sar ; AcSar ; Pro ou l'un de ses dérivés ; Ser ; D-Ser ; Ac-D-Ser ; Thr ; D-Thr ; Ac-D-Thr ; ou un D-aminoacide aromatique qui peut être acylé ;
- A2 est une liaison directe ou un D-aminoacide aromatique ;
- A3 est un L- ou D-aminoacide aromatique ;
- A4 est Ala, Ser, D-Ser, MeSer, Ser($OBu^t$), Ser(OBzl) ou Thr ;
- A5 est un L-aminoacide aromatique ou un L- ou D- aminoacide basique ;
- A6 est Gly ; (S)-spirolactame-Pro ; D-Pro ; D-Ser ; D-Thr ; D-Cys ; D-Met ; D-Asn ; D-Pen ; D-(S-Me)Pen ; D-(S-Et)Pen ; D-Ser($OBu^t$) ; D-Asp($OBu^t$) ; D-Glu($O-Bu^t$) ; D-Thr($O-Bu^t$) ; D-Cys($O-Bu^t$) ; D-Ser($O-R_1$) où $R_1$ est une entité glucidique ; un D-aminoacide aliphatique avec une chaîne latérale alkyle en $C_1$-$C_8$ ou cycloalkyle en $C_3$-$C_6$ ; un D-aminoacide aromatique ; D-cyclohexadiényl-Gly ; D-perhydronaphtyl-Ala ; D-perhydrodiphényl-Ala ; ou un L- ou D-aminoacide basique ;
- A7 est un L-aminoacide aliphatique, linéaire, ramifié ou cyclique de 3 à 20 atomes de carbone qui peut être N-alpha-substitué par un groupe alkyle en $C_1$-$C_4$ éventuellement substitué par un ou plusieurs atomes de fluor ;
- A8 est un L- ou D-aminoacide basique ;
- Z est $GlyNH_2$ ou $D-AlaNH_2$.

**10.** Utilisation selon la revendication 9, où l'analogue peptidique a la formule (SEQ ID N° : 7):

Ac-D-Nal-D-pClPhe-D-Pal-Ser-A5-A6-A7-A8-Pro-D-$AlaNH_2$ (II')

où :

- A5 est Tyr, HTyr, MeTyr, MeHTyr, NicLys ou IprLys ;
- A6 est (S)-spirolactame-Pro, D-Arg, D-NicLys, D-IprLys, D-Cit, D-HCit ou D-Asn ;
- A7 est Leu, MeLeu, Npg ou MeNpg ;
- A8 est Arg, NicLys ou IprLys.

**11.** Utilisation selon la revendication 9 ou 10, où l'analogue peptidique est choisi dans le groupe consistant en l'antide, le [$Npg^7$]-antide, le cétrorélix, le [$Npg^7$]-cétrorélix, l'abarélix et le [$Npg^7$]-abarélix.

**12.** Utilisation selon l'une des revendications 1 à 11, où le dérivé d'α-cyclodextrine est choisi dans le groupe consistant en l'α-cyclodextrine méthylée, l'hexakis(2,3,6-tri-O-méthyl)-α-cyclodextrine, l'α-cyclodextrine carboxyméthylée et l'α-cyclodextrine phosphatée.

**13.** Utilisation selon la revendication 12 où le dérivé d'α-cyclodextrine est l'hexakis(2,3,6-tri-O-méthyl)-α-cyclodextrine.

**14.** Composition pharmaceutique pour la délivrance gastro-intestinale par administration orale d'un analogue peptidique de la LH-RH qui comprend une quantité thérapeutiquement efficace dudit analogue peptidique en combinaison avec l'α-cyclodextrine ou un dérivé de celle-ci.

**15.** Composition pharmaceutique selon la revendication 14, qui comprend en outre des excipients appropriés à la délivrance gastro-intestinale de l'analogue peptidique.

**16.** Composition pharmaceutique selon la revendication 14 ou 15,
où ledit analogue peptidique a la formule (SEQ ID N° : 1):

A1-A2-A3-A4-A5-A6-A7-A8-Pro-Z (A)

où:

- A1 est pGlu ; D-pGlu ; Sar ; AcSar; Pro ou l'un de ses dérivés ; Ser ; D-Ser ; Ac-D-Ser ; Thr ; D-Thr ; Ac-D-Thr ; ou un D-aminoacide aromatique qui peut être acylé ;
- A2 est une liaison directe ; His ; ou un D-aminoacide aromatique ;
- A3 est un L- ou D-aminoacide aromatique ;
- A4 est Ala, Ser, D-Ser, MeSer, Ser($OBu^t$), Ser(OBzl) ou Thr ;
- A5 est un L-aminoacide aromatique ou un L- ou D- aminoacide basique ;
- A6 est Gly ; (S)-spirolactame-Pro ; D-Pro ; D-Ser ; D-Thr ; D-Cys ; D-Met ; D-Asn ; D-Pen ; D-(S-Me)Pen ; D-(S-Et)Pen ; D-Ser($OBu^t$) ; D-Asp($OBu^t$) ; D-Glu($OBu^t$) ; D-Thr($OBu^t$) ; D-Cys($OBu^t$) ; D-Ser($OR_1$) où $R_1$ est une entité glucidique ; un aza-aminoacide ; D-His qui peut être substitué sur le cycle imidazole par un groupe alkyle en $C_1$-$C_6$, acyle en $C_2$-$C_7$ ou benzyle ; un D-aminoacide aliphatique avec une chaîne latérale alkyle en $C_1$-$C_8$ ou cycloalkyle en $C_3$-$C_6$; un D-aminoacide aromatique ; D-cyclohexadiényl-Gly ; D-perhydronaphtyl-Ala; D-perhydrodiphényl-Ala; ou un L- ou D-aminoacide basique ;
- A7 est un L-aminoacide aliphatique, linéaire, ramifié ou cyclique de 3 à 20 atomes de carbone qui peut être N-alpha-substitué par un groupe alkyle en $C_1$-$C_4$ éventuellement substitué par un ou plusieurs atomes de fluor ;
- A8 est un L- ou D-aminoacide basique ;
- Z est $GlyNH_2$ ; D-$AlaNH_2$ ; aza$GlyNH_2$ ; ou un groupe -$NHR_2$ où $R_2$ est un alkyle en $C_1$-$C_4$ qui peut être substitué par un hydroxyle ou un ou plusieurs atomes de fluor ; un cycloalkyle en $C_3$-$C_6$; ou un radical hétérocyclique choisi parmi morpholinyle, pyrrolidinyle et pipéridyle.

**17.** Composition pharmaceutique selon la revendication 16, où ledit analogue peptidique a la formule (SEQ ID N° : 2):

A1-His-A3-A4-A5-A6-A7-A8-Pro-Z (I)

où:

- A1 est pGlu, Sar ou AcSar ;
- A3 est un L-aminoacide aromatique ;
- A4 est Ala, Ser, D-Ser, MeSer, Ser($OBu^t$), Ser(OBzl) ou Thr ;
- A5 est un L-aminoacide aromatique ;
- A6 est Gly ; D-Pro ; (S)-spirolactame-Pro ; D-Ser ; D-Thr ; D-Cys ; D-Met; D-Pen; D-(S-Me)Pen; D-(S-Et)Pen ; D-Ser($OBu^t$) ; D-Asp($OBu^t$) ; D-Glu($OBu^t$) ; D-Thr($OBu^t$) ; D-Cys($OBu^t$) ; D-Ser($OR_1$) où $R_1$ est une entité glucidique; un aza-aminoacide; D-His qui peut être substitué sur le cycle imidazole par un alkyle en $C_1$-$C_6$ ou un groupe benzyle; un D-aminoacide aliphatique avec une chaîne latérale alkyle en $C_1$-$C_8$ où cycloalkyle en $C_3$-$C_6$ ; un D-aminoacide aromatique ; D-cyclohexadiényl-Gly ; D-perhydronaphtyl-Ala ; D-perhydrodiphényl-Ala ; ou un D-aminoacide basique ;
- A7 est un L-aminoacide aliphatique, linéaire, ramifié ou cyclique de 3 à 20 atomes de carbone qui peut être N-alpha-substitué par un groupe alkyle en $C_1$-$C_4$ éventuellement substitué par un ou plusieurs atomes de fluor ;
- A8 est un L-aminoacide basique ;
- Z est $GlyNH_2$ ; aza$GlyNH_2$ ; ou un groupe -$NHR_2$ où $R_2$ est un alkyle en $C_1$-$C_4$ qui peut être substitué par un hydroxyle ou un ou plusieurs atomes de fluor; un cycloalkyle en $C_3$-$C_6$ ; ou un radical hétérocyclique choisi parmi morpholinyle, pyrrolidinyle et pipéridyle.

**18.** Composition pharmaceutique selon la revendication 17, où ledit analogue peptidique a la formule (SEQ ID N° : 3) :

pGlu-His-A3-Ser-A5-A6-A7-Arg-Pro-Z (II)

où A7 est Leu, Tle, Nle, Hol, Npg, Cha ou Ada, qui peut être N-alpha-substitué par un groupe méthyle ou éthyle éventuellement substitué par un ou plusieurs atomes de fluor.

**19.** Composition pharmaceutique selon la revendication 17, où ledit analogue peptidique a la formule (SEQ ID N° : 4):

pGlu-His-A3-Ser-A5-A6-A7-Arg-Pro-Z (III)

où

- A3 et A5 sont chacun indépendamment Phe, Tyr, Trp, 2MeTrp, HPhe, HTyr, Nal, 1Nal, Bal, Pal, 4Pal ou pClPhe;
- A6 est (S)-spirolactame-Pro ; Gly; D-Pro ; D-Ser($OBu^t$); D-Asp($OBu^t$) ; D-Glu($OBu^t$) ; D-Thr($OBu^t$) ; D-Cys ($OBu^t$); D-His ou D-His(Bzl); D-Ala, D-Leu, D-Tle, D-Nle, D-Hol, D-Npg ou D-Cha ; D-Phe, D-HPhe, D-Tyr, D-HTyr, D-Trp, D-2MeTrp, D-Nal, D-1Nal, D-Bal, D-Pal, D-4Pal ou D-pClPhe; D-cyclohexadiényl-Gly, D-perhydronaphtyl-Ala ; D-perhydrodiphényl-Ala ou D-APhe éventuellement substitué par un groupe aminotriazolyle;
- A7 est Leu, Npg ou Cha, qui peut étre N-alpha-substitué par un groupe méthyle ;
- Z est $GlyNH_2$ ; $azaGlyNH_2$ ou $-NHC_2H_5$.

**20.** Composition pharmaceutique selon la revendication 17, où ledit analogue peptidique a la formule (SEQ ID N° : 5):

pGlu-His-Trp-Ser-Tyr-A6-A7-Arg-Pro-Z (IV)

où :

- A6 est (S)-spirolactame-Pro; D-Leu, D-Ala, D-Nal, D-Phe, D-Ser($OBu^t$) ou D-Trp ;
- A7 est Leu, MeLeu, Npg ou MeNpg ;
- Z est $GlyNH_2$ ; $azaGlyNH_2$ ou $-NHC_2H_5$.

**21.** Composition pharmaceutique selon l'une des revendications 17 à 20, où l'analogue peptidique est choisi dans le groupe consistant en la leuproréline, la [$Npg^7$]-leuproréline, la triptoréline, la [$Npg^7$]-triptoréline, la goséréline, la [$Npg^7$]-goséréline, la buséréline et la [$Npg^7$]-buséréline.

**22.** Composition pharmaceutique selon la revendication 21, où l'analogue peptidique est la leuproréline ou la [$Npg^7$]-leuproréline.

**23.** Composition pharmaceutique selon la revendication 16, où ledit analogue peptidique a la formule (SEQ ID N° : 6):

A1-A2-A3-A4-A5-A6-A7-A8-Pro-Z (I')

où:

- A1 est pGlu ; D-pGlu ; Sar ; AcSar; Pro ou l'un de ses dérivés ; Ser ; D-Ser ; Ac-D-Ser ; Thr ; D-Thr ; Ac-D-Thr ; ou un D-aminoacide aromatique qui peut être acylé ;
- A2 est une liaison directe ou un D-aminoacide aromatique ;
- A3 est un L- ou D-aminoacide aromatique ;
- A4 est Ala, Ser, D-Ser, MeSer, Ser($OBu^t$), Ser(OBzl) ou Thr ;
- A5 est un L-aminoacide aromatique ou un L- ou D- aminoacide basique ;
- A6 est Gly ; (S)-spirolactame-Pro ; D-Pro ; D-Ser ; D-Thr ; D-Cys ; D-Met ; D-Asn ; D-Pen ; D-(S-Me)Pen; D-(S-Et)Pen; D-Ser($OBu^t$) ; D-Asp($OBu^t$) ; D-Glu($OBu^t$) ; D-Thr($OBu^t$) ; D-Cys($OBu^t$) ; D-Ser($OR_1$) où $R_1$ est une entité glucidique ; un D-aminoacide aliphatique avec une chaîne latérale alkyle en $C_1$-$C_8$ ou cycloalkyle en $C_3$-$C_6$ ; un D-aminoacide aromatique ; D-cyclohexadiényl-Gly ; D-perhydronaphtyl-Ala ; D-perhydrodiphényl-Ala ; ou un L- ou D-aminoacide basique ;
- A7 est un L-aminoacide aliphatique, linéaire, ramifié ou cyclique de 3 à 20 atomes de carbone qui peut être N-alpha-substitué par un groupe alkyle en $C_1$-$C_4$ éventuellement substitué par un ou plusieurs atomes de fluor ;
- A8 est un L- ou D-aminoacide basique ;
- Z est $GlyNH_2$ ou $D-AlaNH_2$.

**24.** Composition pharmaceutique selon la revendication 23, où l'analogue peptidique a la formule (SEQ ID N° : 7):

Ac-D-Nal-D-pClPhe-D-Pal-Ser-A5-A6-A7-A8-Pro-$D-AlaNH_2$ (II')

où:

- A5 est Tyr, HTyr, MeTyr, MeHTyr, NicLys ou IprLys ;
- A6 est (S)-spirolactame-Pro, D-Arg, D-NicLys, D-IprLys, D-Cit, D-HCit ou D-Asn;
- A7 est Leu, MeLeu, Npg ou MeNpg ;
- A8 est Arg, NicLys ou IprLys.

**25.** Composition pharmaceutique selon la revendication 23 ou 24, où l'analogue peptidique est choisi dans le groupe consistant en l'antide, le [$Npg^7$]-antide, le cétrorélix, le [$Npg^7$]-cétrorélix, l'abarélix et le [$Npg^7$]-abarélix.

**26.** Composition pharmaceutique selon l'une des revendications 14 à 25, où le dérivé d'α-cyclodextrine est choisi dans le groupe consistant en l'α-cyclodextrine méthylée, l'hexakis(2,3,6-tri-O-méthyl)-α-cyclodextrine, l'α-cyclodextrine carboxyméthylée et l'α-cyclodextrine phosphatée.

**27.** Composition pharmaceutique selon la revendication 26 où le dérivé d'α-cyclodextrine est l'hexakis(2,3,6-tri-O-méthyl)-α-cyclodextrine.

**28.** Composition pharmaceutique selon l'une des revendications 14 à 27, qui comprend en outre un inhibiteur de protéase et/ou un agent augmentant l'absorption.

FIG.1

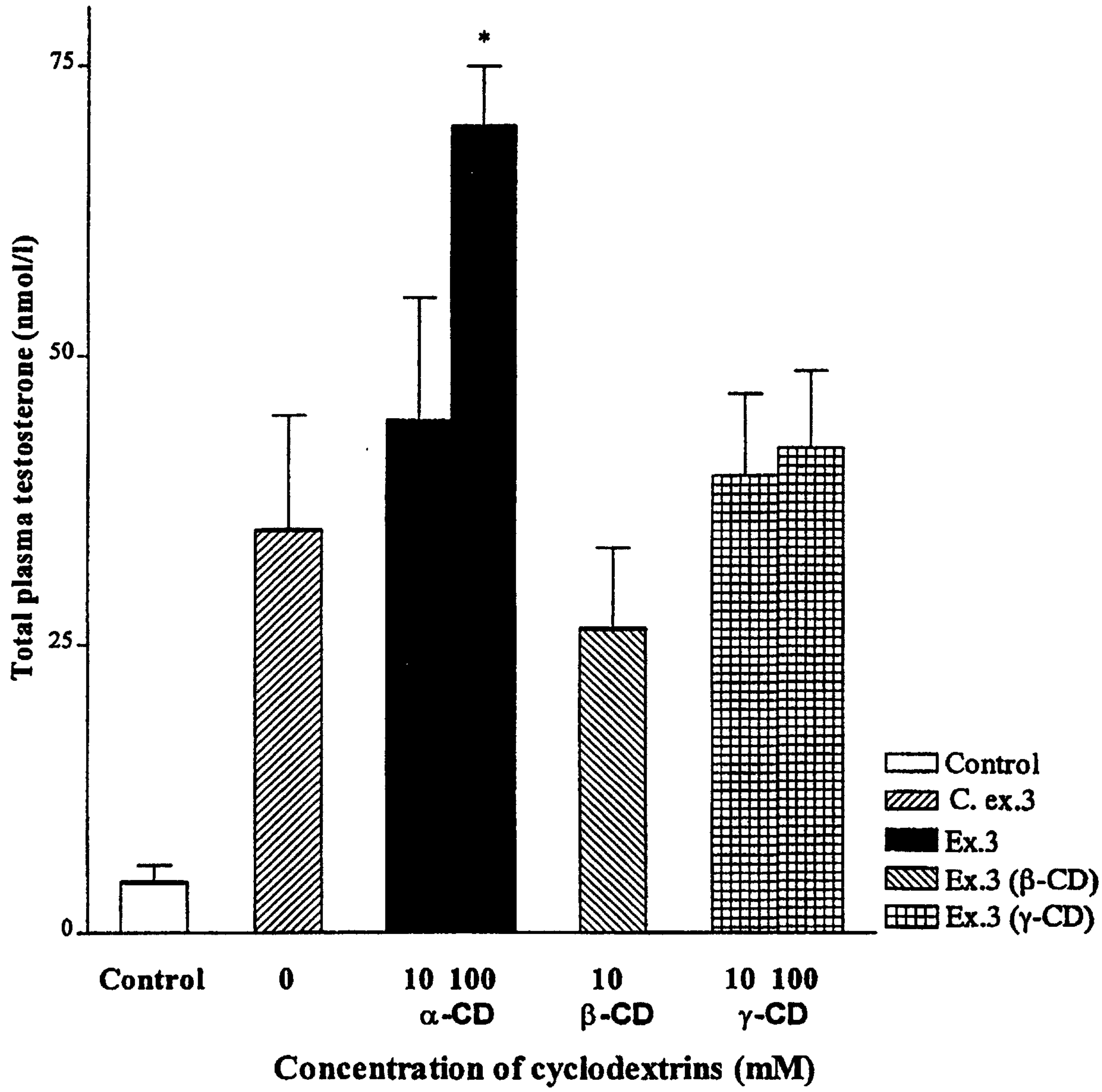


*p < 0.05 vs C. ex.3 in vehicle

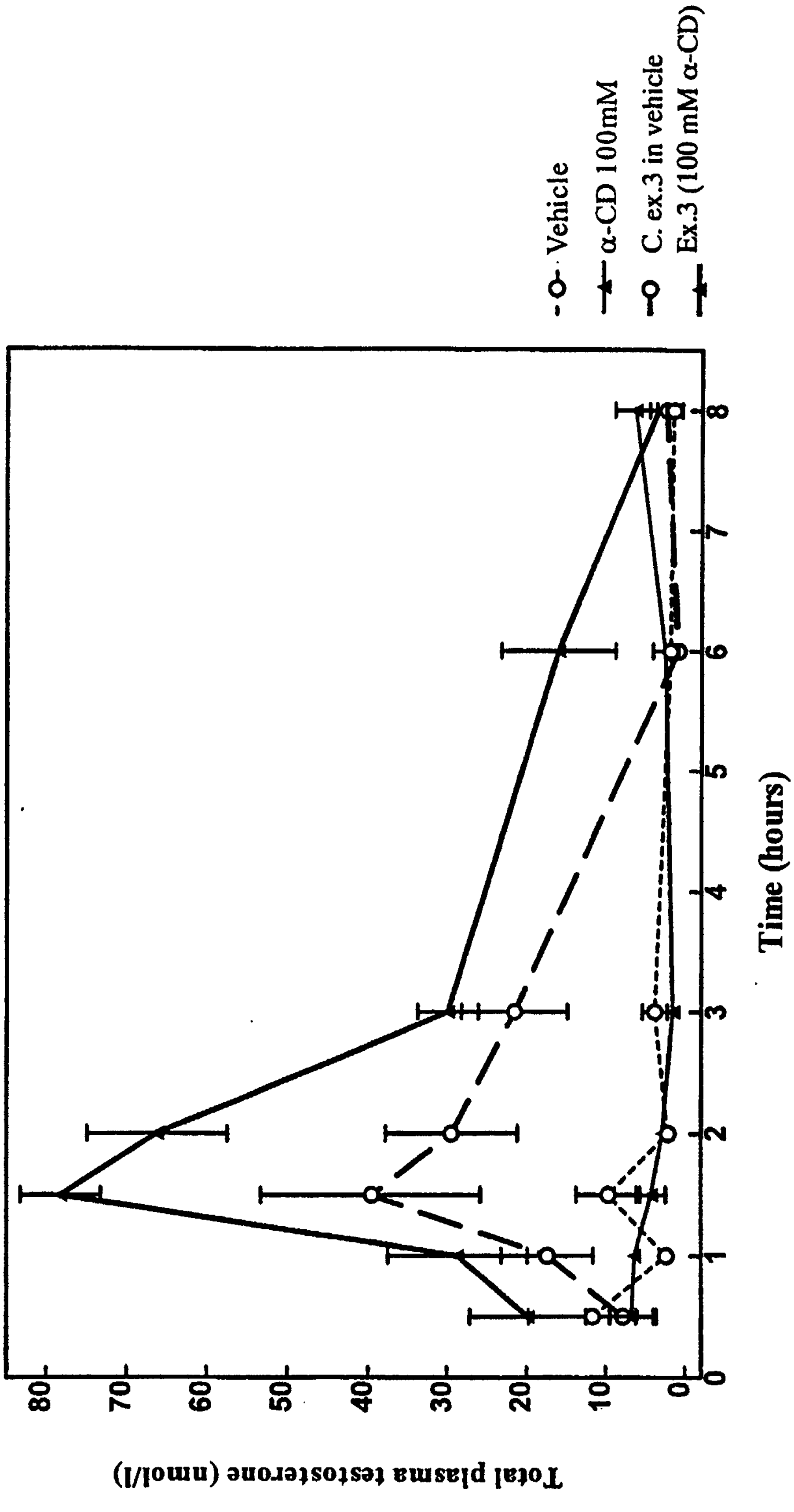
Total plasma testosterone (nmol/l)
0
10
20
30
40
50
60
70
80
Time (hours)
0
1
2
3
4
5
6
7
8
Vehicle
α-CD 100mM
C. ex.3 in vehicle
Ex.3 (100 mM α-CD)

FIG. 2

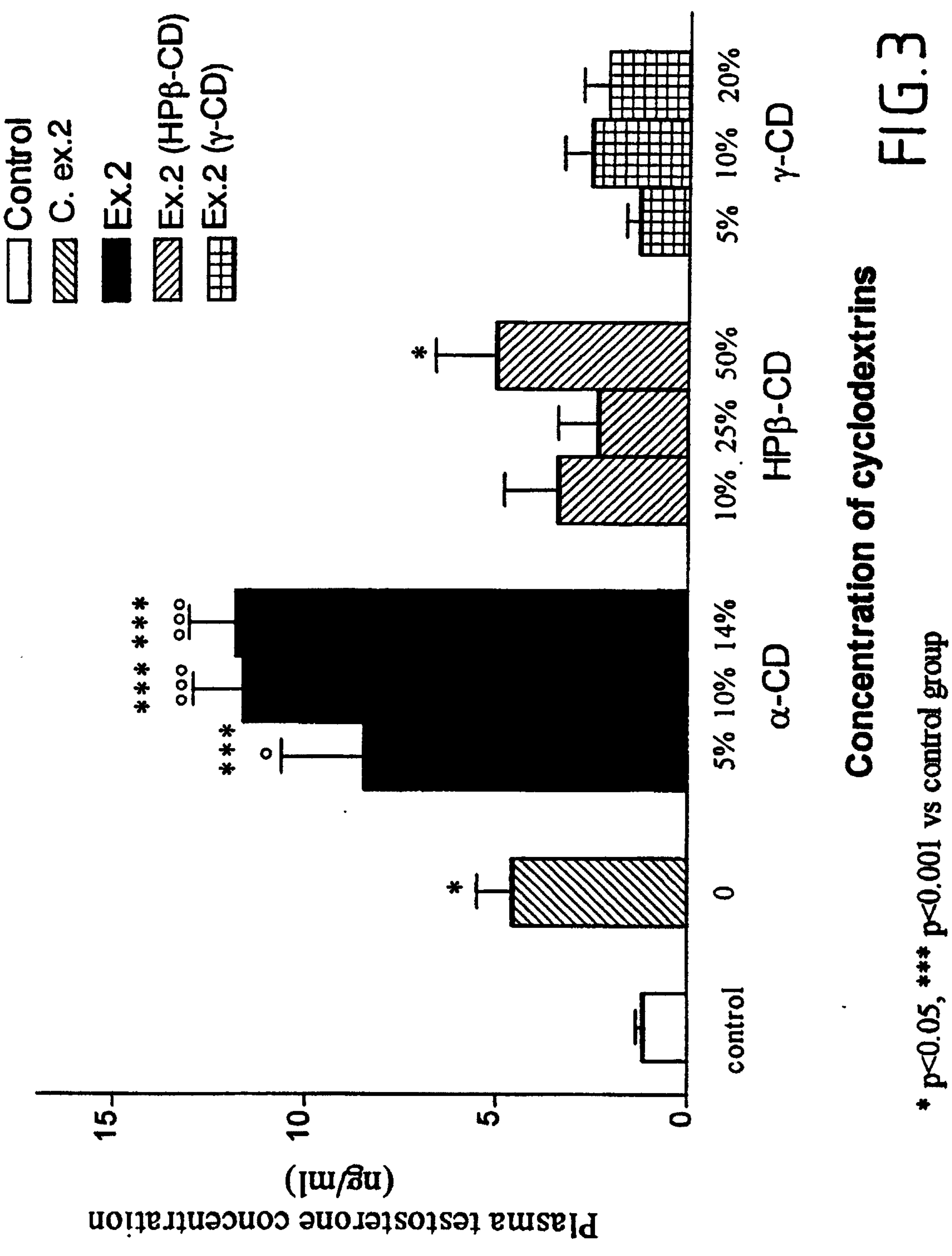


FIG. 3

* $p<0.05$, *** $p<0.001$ vs control group
° $p<0.05$, °°° $p<0.001$ vs C. ex.2 in vehicle

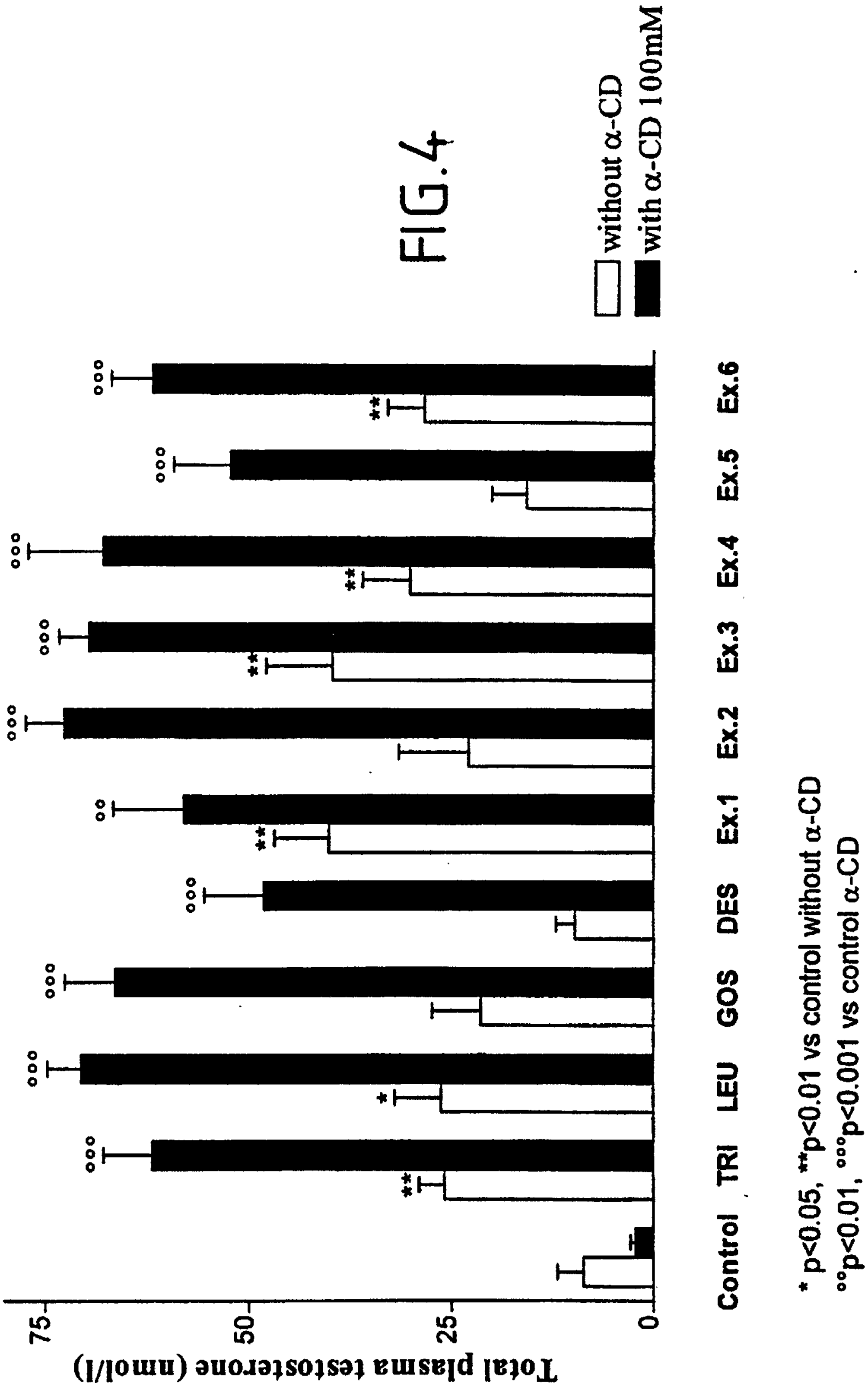
Total plasma testosterone (nmol/l)
75
50
25
0
Control
TRI
LEU
GOS
DES
Ex.1
Ex.2
Ex.3
Ex.4
Ex.5
Ex.6
without α-CD
with α-CD 100mM
* p<0.05, **p<0.01 vs control without α-CD
°°p<0.01, °°°p<0.001 vs control α-CD

FIG. 4

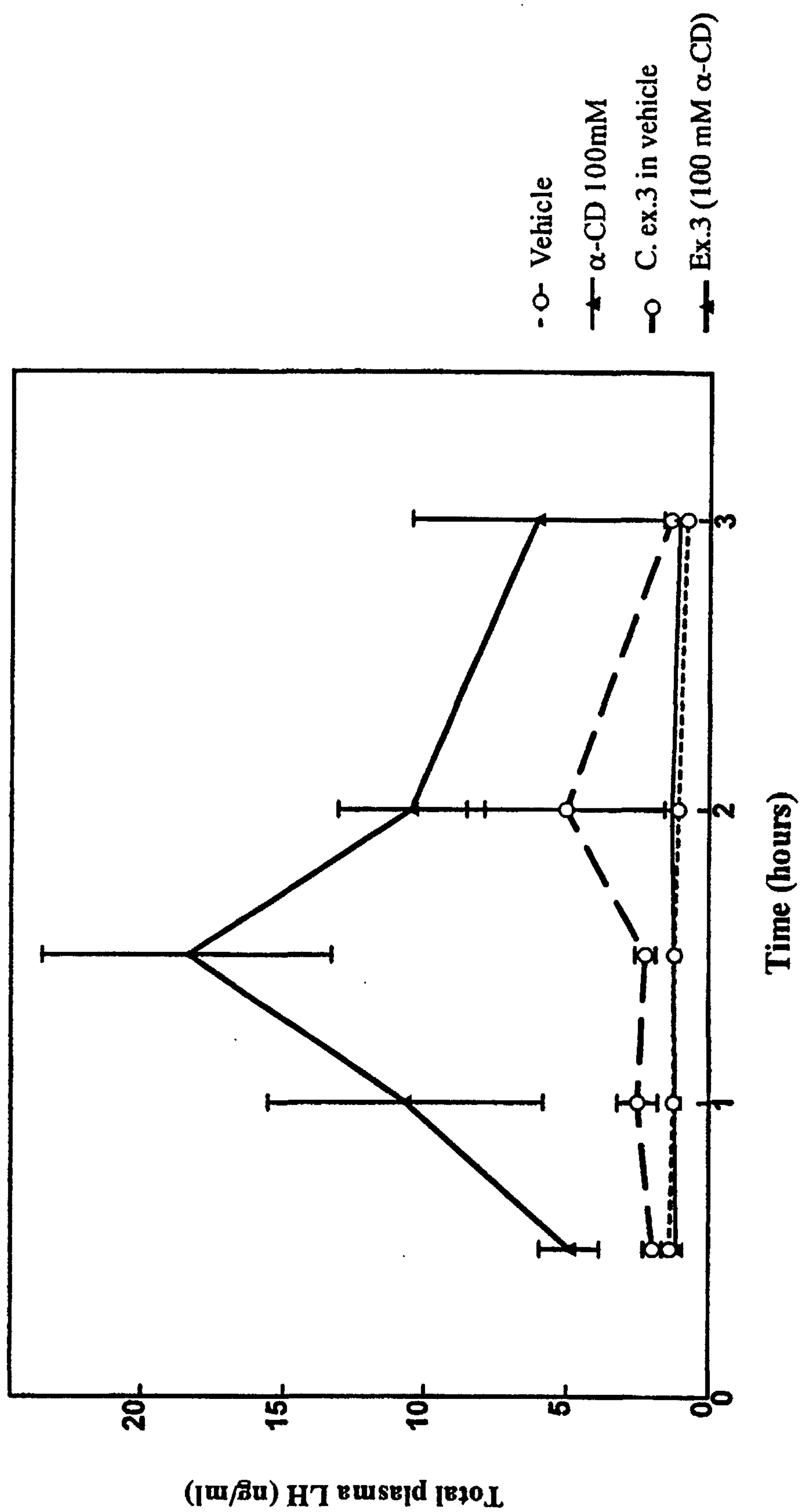
Vehicle
α-CD 100mM
C. ex.3 in vehicle
Ex.3 (100 mM α-CD)
Total plasma LH (ng/ml)
0
5
10
15
20
0
1
2
3
Time (hours)

FIG.5

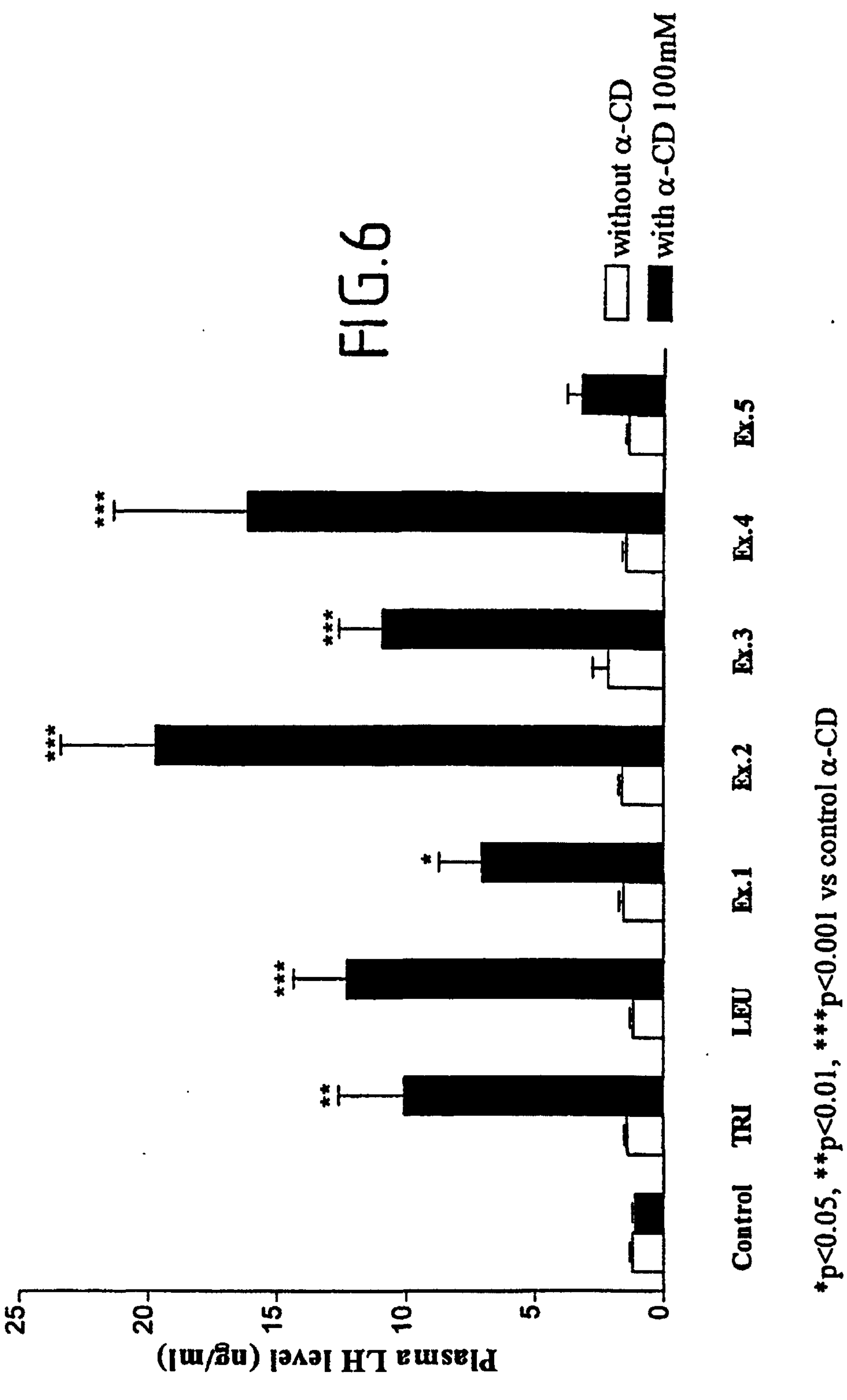

Plasma LH level (ng/ml)
25
20
15
10
5
0
Control
TRI
LEU
Ex.1
Ex.2
Ex.3
Ex.4
Ex.5
**
***
*
***
***
***
without α-CD
with α-CD 100mM
*p<0.05, **p<0.01, ***p<0.001 vs control α-CD


FIG.6

FIG.7

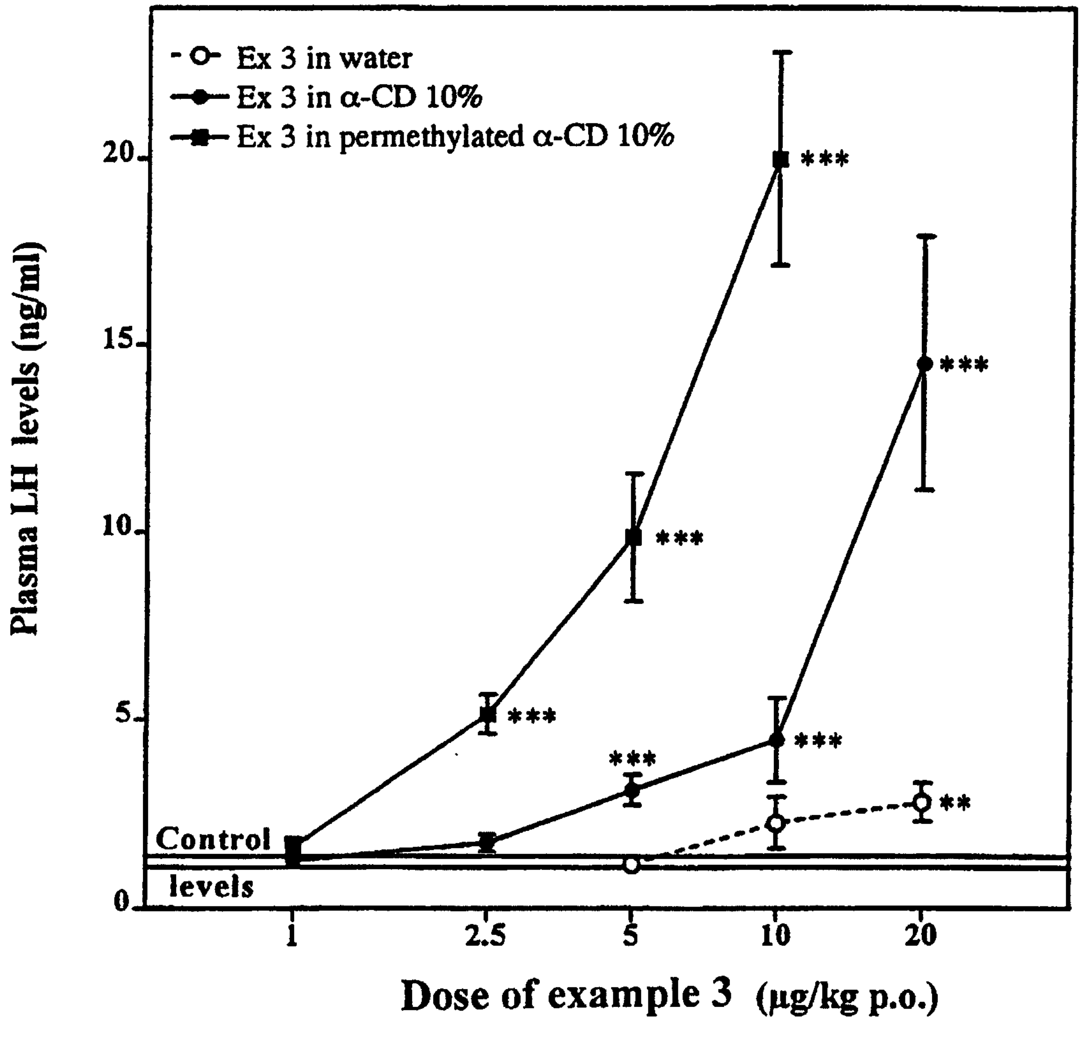


(**p<0.01;***p<0.001 vs. control unstimulated LH levels)